(19) Europäisches Patentamt / European Patent Office / Office européen des brevets

(11) **EP 2 297 205 B1**

(12) **FASCICULE DE BREVET EUROPEEN**

(45) Date de publication et mention de la délivrance du brevet:
**25.12.2013 Bulletin 2013/52**

(51) Int Cl.:
***C07K 16/28*** *(2006.01)*

(21) Numéro de dépôt: **09772761.4**

(22) Date de dépôt: **06.07.2009**

(86) Numéro de dépôt international:
**PCT/FR2009/051335**

(87) Numéro de publication internationale:
**WO 2010/001079 (07.01.2010 Gazette 2010/01)**

(54) **UTILISATION DE LA PROTEINE IRAP POUR LA MISE EN OEUVRE DE METHODES DE DIAGNOSTIC E DE PRONOSTIC**

VERFAHREN ZUR IMPLEMENTIERUNG VON DIAGNOSE- UND PROGNOSEVERFAHREN MIT DEM PROTEIN IRAP

USE OF THE IRAP PROTEIN FOR IMPLEMENTING METHODS OF DIAGNOSIS AND OF PROGNOSIS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **04.07.2008 FR 0803802**

(43) Date de publication de la demande:
**23.03.2011 Bulletin 2011/12**

(73) Titulaire: **Université Joseph Fourier**
**38041 Grenoble Cedex 09 (FR)**

(72) Inventeur: **BOTTARI, Serge**
**F-38330 Biviers (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
- **YAMAHARA N. ET AL: "Placental leucine aminopeptidase/oxytocinase in maternal serum and placental during normal pregnency" LIFE SCIENCE, vol. 66, no. 15, 2000, pages 1401-1410, XP002512633**
- **TSUJIMOTO M ET AL: "The oxytocinase subfamily of M1 aminopeptidases" BIOCHIMICA ET BIOPHYSICA ACTA (BBA) - PROTEINS & PROTEOMICS, ELSEVIER, vol. 1751, no. 1, 1 août 2005 (2005-08-01), pages 9-18, XP004995172 ISSN: 1570-9639**
- **ALBISTON ET AL: "Therapeutic targeting of insulin-regulated aminopeptidase: Heads and tails?" PHARMACOLOGY AND THERAPEUTICS, ELSEVIER, GB, vol. 116, no. 3, 20 novembre 2007 (2007-11-20), pages 417-427, XP022354101 ISSN: 0163-7258**
- **IWASE A. ET AL.: "Characterization of a secretase activity for placental leucine aminopeptidase" ARCHIVES OF BIOCHEMISTRY AND BIOPHYSICS, vol. 393, no. 1, 10 août 2001 (2001-08-10), pages 163-169, XP002512634**

**Description**

L'invention concerne l'utilisation de la protéine IRAP pour la mise en oeuvre de méthodes de diagnostic et de pronostic.

La protéine IRAP (Insulin-Regulated AminoPeptidase ; EC 3.4.11.3) également connue sous le nom de Placental Leucine AminoPeptidase (P-LAP) et de Leucine-cystinyl aminopeptidase (L-CAP) est une protéine métalloprotéinase à zinc transmembranaire qui existe sous trois isoformes (Swiss-Prot: Q9UIQ6 :1, 2 et 3; représentées respectivement par les SEQ ID NO: 1 à 3).

Lorsqu'elle est insérée au niveau de la membrane plasmique son domaine extracellulaire peut être clivé et sécrété.

La partie sécrétée d'une isoforme non-spécifiée de la protéine présumée être IRAP a été dosée dans le sang par une méthode enzymatique en utilisant un substrat synthétique non spécifique, la L-leucine-nitroanilide en présence de méthionine (Mizutani, S., Yoshino, M., and Oya, M. (1976) Clin Biochem 9(1), 16-18).

Utilisant cette méthode Mizutani, Oya et Tomoda ont mis en évidence une cystinyl-leucine aminopeptidase, dans le sérum de femmes enceintes, dont la concentration augmente au cours de la grossesse (Yamahara, N., Nomura, S., Suzuki, T., Itakura, A., Ito, M., Okamoto, T., Tsujimoto, M., Nakazato, H., and Mizutani, S: (2000) Life Sci 66(15), 1401-1410). Cette aminopeptidase est essentiellement d'origine placentaire, raison pour laquelle elle a été appelée P-LAP (Tsujimoto, M., Mizutani, S., Adachi, H., Kimura, M., Nakazato, H., and Tomoda, Y. (1992) Arch Biochem Biophys 292(2), 388-392) et correspond au domaine sécrété de la protéine.

Cette enzyme dégrade l'oxytocine (Naruki, M., Mizutani, S., Goto, K., Tsujimoto, M., Nakazato, H., Itakura, A., Mizuno, K., Kurauchi, O.; Kikkawa, F., and Tomoda, Y. (1996) Peptides 17(2), 257-261), la vasopressine (Wallis, M. G., Lankford, M. F., and Keller, S. R. (2007) Am J Physiol Endocrinol Metab 293(4), E1092-1102), l'angiotensine II et III (Matsumoto, H., Rogi, T., Yamashiro, K., Kodama, S., Tsuruoka, N., Hattori, A., Takio, K., Mizutani, S., and Tsujimoto, M: (2000) Eur J Biochem 267(1), 46-52) ainsi qu'une série d'autres peptides (Albiston, A. L., Peck, G. R., Yeatman, H. R., Fernando, R., Ye, S., and Chai, S. Y. (2007) Pharmacol Ther 116(3), 417-427). Les concentrations sériques de cette aminopeptidase n'ont jamais été rapportées chez l'homme ni chez la femme non gravite.

A l'occasion de son clonage, il est apparu que P-LAP correspond à la protéine IRAP ainsi qu'au récepteur de l'angiotensine IV (Keller, S. R., Scott, H. M., Mastick, C. C., Aebersold, R., and Lienhard, G. E. (1995) J Biol Chem 270 (40), 23612-23618; Rogi, T., Tsujimoto, M., Nakazato, H., Mizutani, S., and Tomoda, Y. (1996) J Biol Chem 271(1), 56-61; Albiston, A. L., McDowall, S. G., Matsacos, D., Sim, P., Clune, E., Mustafa, T., Lee, J., Mendelsohn, F. A., Simpson, R. J., Connolly, L. M., and Chai, S. Y. (2001) J Biol Chem 276(52), 48623-48626).

La demande de brevet WO 2005/038462 décrit un réactif pour le diagnostic et/ou une évaluation pronostique de carcinomes qui comprend un anticorps polyclonal and-P-LAP, obtenu par immunisation avec la protéine P-LAP entière. Compte tenu de la forte homologie entre les différentes aminopéptidases, l'anticorps n'est vraisemblablement pas spécifique pour IRAP et devrait aussi reconnaître d'autres aminopeptidases. Il ne devrait donc pas permettre de diagnostiquer une pathologie liée de façon précise à une modification de l'expression ou de la concentration plasmatique d'IRAP.

GLUT4 est le transporteur de glucose qui permet la captation du glucose circulant par les muscles et le tissu adipeux en réponse à l'insuline. Dans des conditions non stimulées (conditions basales), GLUT4 est efficacement retenu à l'intérieur de la cellule dans des compartiments (vésicules) intracellulaires, par un mécanisme de rétention encore inconnu. En réponse à la stimulation insulinique, GLUT4 est transporté puis inséré à la membrane plasmique par une translocation accrue, permettant ainsi la captation cellulaire du glucose.

IRAP co-localise avec le transporteur du glucose GLUT4 et est co-transloquée avec celui-ci de façon stoechiométrique (Keller, S. R (2004) Biol Pharm Bull 27(6), 761-764). Cette translocation vers la membrane plasmique est stimulée par l'insuline de la même façon que celle de GLUT4 (Karylowski, O., Zeigerer, A., Cohen, A., and McGraw, T. E. (2004) Mol Biol Cell 15(2), 870-882; Subtil, A., Lampson, M. A., Keller, S. R., and McGraw, T. E. (2000) J Biol Chem 275(7), 4787-4795).

Par ailleurs l'expression d'IRAP conditionne l'expression de GLUT4 car chez les animaux transgéniques IRAP$^{-/-}$, les taux de GLUT4 sont réduits de 50 à 80 % (Keller, S. R., Davis, A. C., and Clairmont, K. B. (2002) J Biol Chem 277 (20), 17677-17686).

Chez le diabétique de type 2 on retrouve à la fois une réduction de l'expression et de la translocation de GLUT4 vers la membrane plasmique dans le muscle et le tissu adipeux (Kahn, B. B. (1992) J Clin Invest 89(5), 1367-1374).

De même et bien que les taux cellulaires d'IRAP ne soient pas modifiés, sa translocation est également diminuée dans le muscle et le tissu adipeux des diabétiques de type 2 (Garvey, W. T., Maianu, L., Zhu, J. H., Brechtel-Hook, G., Wallace, P., and Baron, A. D. (1998) J Clin Invest 101(11), 2377-2386; Maianu, L., Keller, S. R., and Garvey, W. T. (2001) J Clin Endocrinol Metab 86(11), 5450-5456).

Par ailleurs, il a été montré une relation entre la protéine IRAP et le développement de la chimiorésistance aux médicaments anticancéreux (Kondo C. et al., Int J. Cancer, 118, 1390-1394, 2006). Selon cet article, IRAP réduit en effet la sensibilité aux médicaments anticancéreux en inhibant l'expression du facteur de déclenchement de l'apoptose et augmente l'expression du facteur d'inhibition de l'apoptose.

Le domaine extracellulaire d'IRAP est clivé par des métalloprotéases appartenant vraisemblablement à la famille des ADAM dont ADAM9 (SwissProt Q13443) et ADAM12 (SwissProt 043184) (Ito, N., Nomura, S., Iwase, A., Ito, T., Kikkawa, F., Tsujimoto, M., Ishiura, S., and Mizutani, S. (2004) Biochem Biophys Res Commun 314(4), 1008-1013) et relargué dans la circulation sanguine. ADAM9 (MDC9) est exprimée dans différents tissus dont le muscle squelettique et le tissu adipeux (Hotoda, N., Koike, H., Sasagawa, N., and Ishiura, S. (2002) Biochem Biophys Res Commun 293 (2), 800-805) et ADAM12 est essentiellement exprimée dans le muscle. Plusieurs autres membres de cette famille sont également exprimés dans le muscle et le tissu adipeux.

A ce jour, les seules relations décrites entre la concentration du domaine extracellulaire d'IRAP dans un milieu biologique et une pathologie concernent la prééclampsie sévère ainsi que la menace d'accouchement prématuré. Dans ces deux pathologies les concentrations de P-LAP circulante sont inférieures à celles observées chez les femmes témoins d'âge gestationnel équivalent. Cependant, les méthodes décrites dans l'art antérieur sont basées sur le dosage enzymatique, au moyen d'un substrat non specifique, du domaine extracellulaire d'IRAP.

Aussi, il existe un réel besoin de fournir une méthode fiable et specifique permettant de doser le domaine extracellulaire d'IRAP circulant.

Un des buts de la présente invention est de fournir une méthode de dosage *in vitro* de la concentration en protéine IRAP dans du sérum ou du plasma ou des tissus d'un mammifère, spécifiquement du domaine extracellulaire de la protéine IRAP.

Un autre but de l'invention est de fournir un anticorps monoclonal spécifique du domaine extracellulaire des différentes isoformes de la protéine IRAP.

Un autre but de l'invention est de fournir une méthode de diagnostic de pathologies dans lesquelles un excès ou une baisse de la concentration en protéine IRAP est impliqué.

Un dernier but est de fournir un anticorps monoclonal spécifique du domaine extracellulaire des différentes isoformes de la protéine IRAP et inhibiteur de leur activité enzymatique dans une optique thérapeutique.

Par conséquent, la présente invention est illustrée par l'utilisation du domaine extracellulaire de la protéine IRAP (« insulin-responsive aminopeptidase »), pour la mise en oeuvre d'une méthode de dosage *in vitro* de la concentration en domaine extracellulaire sécrété de la protéine IRAP dans du sérum, du plasma ou des tissus d'un mammifère, notamment de l'Homme.

L'invention concerne l'utilisation du domaine extracellulaire circulant de la protéine IRAP (« insulin-responsive aminopeptidase »), pour la mise en oeuvre d'une méthode de dosage *in vitro* de la concentration en domaine extracellulaire sécrété de la protéine IRAP dans du sérum ou le plasma d'un mammifère, notamment de l'Homme, ledit dosage étant effectué à l'aide d'un anticorps monoclonal dirigé contre la partie extracellulaire de la protéine IRAP représenté par les SEQ ID NO : 7 à 11.

La protéine IRAP peut avoir différentes dénominations telles que : « Insulin responsive aminopeptidase », « Insulin-regulated membrane aminopeptidase », « Leucyl-cystinyl aminopeptidase (L-CAP) », « Placental leucine aminopeptidase (P-LAP)», « Cystinyl aminopeptidase », « Oxytocinase », « OTase », « Vesicle protein of 165 kDa», « Vp165», ou « GP160 ». Elles correspondent toutes à une ou plusieurs des isoformes de la protéine enregistrée sous les n° Q9UIQ6 1, 2 et 3 de la base de données Swiss-Prot.

Ces différentes dénominations pourront être employées indifféremment dans la suite de la description et désignent la même protéine.

La protéine IRAP, quelles que soient ses isoformes est constituée d'une partie N-terminale d'environ 110 acides aminés présente dans le cytoplasme, d'une partie transmembranaire d'environ 20 acides aminés et une partie C-terminale extracellulaire comportant les acides aminés restants.

Comme indiqué plus haut, le domaine extracellulaire peut être clivé et sécrété.

En conséquence, l'expression « domaine extracellulaire » comprend aussi bien le domaine extracellulaire encore lié à la membrane de la cellule et que l'on trouve par conséquent dans les tissus, que le domaine extracellulaire circulant, c'est-à-dire après clivage et sécrétion, que l'on trouve dans la circulation sanguine.

Par « mammifère », on entend un taxon inclus dans les vertébrés et qui regroupe près de 5400 espèces. Lesdites espèces sont définies dans :Wilson, D. E., and Reeder, D. M. (eds), Mammal Species of the World, Johns Hopkins University Press, 16/11/2005.

L'un des avantages de la méthode de dosage in vitro de l'invention est donc de permettre le dosage spécifique du domaine extracellulaire de la protéine IRAP chez un mammifère et plus particulièrement:

- soit le domaine extracellulaire circulant dans le sérum, ou le plasma
- soit le domaine extracellulaire encore lié à la membrane dans les tissus, y compris les hématies.

Dans un mode de réalisation avantageux, l'invention concerne l'utilisation du domaine extracellulaire de la protéine IRAP, dans laquelle ladite protéine IRAP correspond à l'une de ses isoformes, en particulier les isoformes définies par les SEQ ID N°1 à 3, ou à l'un de ses variants, en particulier les variants définis par les SEQ ID NO:4 à 6, ledit domaine extracellulaire étant représenté par les SEQ ID NO : 7 à 11.

La protéine IRAP peut exister sous au moins trois isoformes (Swiss-Prot: Q9UIQ6 1, 2 et 3; représentées par les SEQ ID NO: 1 à 3 respectivement) ainsi que plusieurs variants de l'isoforme 1 : 031616 (S86P, serine remplacée par une proline en position 86), 012812 (A763T alanine remplacée par une thréonine en position 763) et 031617 (I963V; isoleucine remplacée par une valine en position 963) représentés par les SEQ ID NO : 4 à 6 respectivement.

Le domaine extracellulaire de chaque isoforme 1 à 3 est représenté par les SEQ ID NO : 7 à 9 respectivement et le domaine extracellulaire des variants 031616, 0128412 et 031617 est représenté par les SEQ ID NO :7, 10 et 11.

Dans toute cette description, le terme IRAP peut être employé seul mais inclut également ses isoformes et/ou variants.

Dans un mode de réalisation avantageux, l'invention est illustrée par l'utilisation du domaine extracellulaire de la protéine IRAP définie ci-dessus et/ou de l'une de ses isoformes définie ci-dessus et/ou de l'un de ses variants défini ci-dessus, pour le diagnostic et/ou le pronostic *in vitro,* ou le suivi *in vitro* de pathologies liées à des défauts de translocation du transporteur du glucose GLUT4 ou de protéines associées, incluant IRAP.

L'invention concerne l'utilisation du domaine extracellulaire circulant de la protéine IRAP définie ci-dessus pour le diagnostic et/ou le pronostic *in vitro,* ou le suivi *in vitro* de pathologies liées à des défauts de translocation du transporteur du glucose GLUT4 ou de protéines associées.

Par « diagnostic *in vitro»* il faut entendre le raisonnement menant à l'identification *in vitro* de la cause (l'origine) d'une défaillance, d'un problème ou d'une maladie.

Par « pronostic *in vitro»,* il faut entendre l'appréciation *in vitro* du degré de gravité et de l'évolution ultérieure d'une maladie y compris son issue.

Comme précisé plus haut, IRAP co-localise avec le transporteur du glucose GLUT4 et est co-transloquée avec celui-ci de façon stoechiométrique.

En conséquence, la détermination de la concentration la protéine IRAP ou de l'une de ses isoformes ou de l'un de ses variants défini ci-dessus présente dans le sérum ou le plasma, les hématies ou les tissus est représentative de la translocation et donc de la quantité de transport du glucose.

Un autre avantage de la méthode dé dosage de l'invention est donc la détermination d'un défaut de transport du glucose et par conséquent l'identification de la cause d'une pathologie ou de son degré de gravité ou encore le suivi de la pathologie et de son évolution.

Dans un mode de réalisation avantageux, la méthode de dosage définie ci-dessus permet l'évaluation d'un traitement dirigé contre cette maladie par comparaison des concentrations du domaine extracellulaire de protéine IRAP obtenues avant et après le traitement.

Les pathologies liées au défaut de transport du glucose peuvent être sans être limitées à celles-ci : l'insulino-résistance, le diabète de type 2, le diabète gestationnel. Selon un mode de réalisation plus avantageux, l'invention est illustrée par l'utilisation du domaine extracellulaire de la protéine IRAP définie ci-dessus et/ou de l'une de ses isoformes définie ci-dessus et/ou de l'un de ses variants défini ci-dessus, pour le diagnostic et/ou le pronostic *in vitro* de pathologies associées à la surexpression et/ou à l'augmentation de la translocation à la membrane plasmique de IRAP et/ou de ses isoformes et/ou variants par rapport à un individu sain, ou le suivi *in vitro* de pathologies associées à la surexpression de IRAP et/ou de ses isoformes et/ou variants chez un patient, par rapport à un individu sain.

L'invention concerne l'utilisation du domaine extracellulaire circulant de la protéine IRAP définie ci-dessus pour le diagnostic et/ou le pronostic *in vitro* de pathologies associées à la surexpression et/ou à l'augmentation de la translocation vers la membrane de IRAP et/ou de ses isoformes et/ou variants par rapport à un individu sain, ou le suivi *in vitro* de pathologies associées à la surexpression de IRAP et/ou à l'augmentation de la translocation vers la membrane de IRAP et/ou de ses isoformes et/ou variants chez un patient, par rapport à un individu sain.

Dans le cadre d'une surexpression et/ou à l'augmentation de la translocation à la membrane plasmique de la protéine IRAP, la concentration du domaine extracellulaire de la protéine IRAP, sécrétée ou non, déterminée chez un patient, par la méthode de dosage de l'invention, sera supérieure à celle obtenue chez un individu sain, indiquant ainsi une surexpression de la protéine IRAP.

Dans le cadre du suivi d'une pathologie liée à une surexpression et/ou à l'augmentation de la translocation à la membrane plasmique de la protéine IRAP chez un patient, la diminution de la concentration du domaine extracellulaire de la protéine IRAP déterminée chez ledit patient en cours de traitement par la méthode de dosage de l'invention, par rapport à la concentration du domaine extracellulaire de la protéine IRAP déterminée avant traitement, permettra de montrer l'efficacité du traitement.

A l'inverse, une augmentation ou une stabilisation de la concentration du domaine extracellulaire de la protéine IRAP déterminée chez ledit patient en cours de traitement, par rapport à la concentration du domaine extracellulaire, sécrété ou non, de la protéine IRAP déterminée avant traitement, montrera l'inefficacité du traitement permettant ainsi une modification des doses ou du principe actif utilisé.

Par conséquent, on considère qu'il y a surexpression et/ou à augmentation de la translocation à la membrane plasmique de la protéine IRAP chez un individu lorsque la valeur maximum de la concentration en protéine IRAP, sécrétée ou non, ci-dessus indiquée est augmentée de 25% ou plus chez un patient.

Des exemples de pathologies liées à la surexpression et/ou à l'augmentation de la translocation à la membrane plasmique de la protéine IRAP sans être limités à ceux-ci sont toutes les maladies impliquant un phénomène prolifératif, en particulier les cancers-tels et de façon non exhaustive :l'adénocarcinome ovarien, le cancer de l'endomètre, le choriocarcinome, le cancer du pancréas, le cancer du sein, le cancer de la prostate, le cancer de l'estomac, le cancer du rectum ou les cancers de la sphère ORL.

Selon un mode de réalisation plus avantageux, l'invention est illustrée par l'utilisation du domaine extracellulaire de la protéine IRAP définie ci-dessus et/ou de l'une de ses isoformes définie ci-dessus et/ou de l'un de ses variants défini ci-dessus, pour le diagnostic et/ou le pronostic *in vitro* de pathologies associées à la sous-expression et/ou à la diminution de la translocation à la membrane plasmique de IRAP et/ou de ses isoformes et/ou variants par rapport à un individu sain, ou le suivi *in vitro* de pathologies associées à la sous-expression et/ou à la diminution de la translocation à la membrane plasmique de IRAP et/ou de ses isoformes et/ou variants chez un patient.

L'invention concerne l'utilisation du domaine extracellulaire circulant de la protéine IRAP définie ci-dessus pour le diagnostic et/ou le pronostic *in vitro* de pathologies associées à la sous-expression et/ou à la diminution de la translocation vers la membrane de IRAP et/ou de ses isoformes et/ou variants par rapport à un individu sain, ou le suivi *in vitro* de pathologies associées à la sous-expression et/ou à la diminution de la translocation vers la membrane de IRAP ou de ses isoformes ou variants chez un patient.

Dans le cadre d'une sous-expression et/ou à la diminution de la translocation à la membrane plasmique de la protéine IRAP, la concentration du domaine extracellulaire, sécrété ou non, de la protéine IRAP déterminée chez un patient, par la méthode de dosage de l'invention, sera inférieure à celle obtenue chez un individu sain, indiquant ainsi une sous expression et/ou la diminution de la translocation à la membrane plasmique de la protéine IRAP.

Dans le cadre du suivi d'une pathologie liée à une sous-expression et/ou à la diminution de la translocation à la membrane plasmique de la protéine IRAP chez un patient, l'augmentation de la concentration du domaine extracellulaire de la protéine IRAP déterminée chez ledit patient en cours de traitement par la méthode de dosage de l'invention, par rapport à la concentration du domaine extracellulaire de la protéine IRAP déterminée avant traitement, permettra de montrer l'efficacité du traitement.

A l'inverse, une diminution ou une stabilisation de la concentration du domaine extracellulaire de la protéine IRAP déterminée chez ledit patient en cours de traitement, par rapport à la concentration du domaine extracellulaire de la protéine IRAP déterminée avant traitement, montrera l'inefficacité du traitement permettant ainsi une modification des doses ou du principe actif utilisé.

On considère qu'il y a sous-expression de la protéine IRAP lorsque la valeur minimum de la concentration en protéine IRAP ci-dessus indiquée est diminuée de 25% ou plus.

Dans un mode de réalisation avantageux, l'utilisation du domaine extracellulaire circulant de la protéine IRAP défini ci-dessus, et/ou de l'une de ses isoformes définie ci-dessus et/ou de l'un de ses variants défini ci-dessus, permet le diagnostic et/ou le pronostic *in vitro* de la chimiorésistance aux médicaments anticancéreux.

Par le terme « chimiorésistance », il faut comprendre une sensibilité réduite ou totalement perdue à un traitement anticancéreux lors d'une chimiothérapie par des médicaments anticancéreux, acquise ou inhérente, et qui restreint ou annihile complètement l'efficacité du traitement anticancéreux.

Des exemples de médicaments anticancéreux sans être limités à ceux-ci sont le paclitaxel (Taxol), le carboplatine...

Par conséquent, le dosage du domaine extracellulaire d'IRAP et/ou de l'une de ses isoformes définie ci-dessus, et/ou de l'un de ses variants défini ci-dessus, montrant une surexpression et/ou à l'augmentation de la translocation à la membrane plasmique de la protéine IRAP chez un individu, notamment lorsque la valeur maximum de la concentration en protéine IRAP ci-dessus indiquée est augmentée de 25% ou plus chez un patient, permet le diagnostic et/ou le pronostic de la résistance à la chimiothérapie.

Selon un mode de réalisation avantageux, l'utilisation du domaine extracellulaire de la protéine IRAP définie ci-dessus et/ou de l'une de ses isoformes définie ci-dessus, et/ou de l'un de ses variants défini ci-dessus, permet le diagnostic et/ou le pronostic *in vitro,* ou le suivi *in vitro* de pathologies liées à des défauts de translocation du transporteur du glucose GLUT4 et notamment à la sous expression de IRAP et/ou de ses isoformes et/ou variants, et qui sont parmi celles associées à l'insulinorésistance, le diabète de type 2, le diabète gestationnel.

L'insulino-résistance se traduit par une moins bonne captation du glucose par les tissus sensibles en réponse à l'insuline. Cette maladie évolue vers le diabète de type 2 qui apparaît lorsque le taux de glucose dans le sang (glycémie) dépasse les valeurs normales (110 mg/dL à jeun et 140 mg/dL 2 h après l'ingestion de 75 g de glucose). Cette augmentation du taux de glucose dans le sang hyper-stimule le pancréas, qui augmente la sécrétion d'insuline pour compenser l'augmentation de la glycémie.

Le diabète gestationnel représente tout état d'intolérance au glucose, quelle que soit sa sévérité, apparu au cours de la grossesse chez une femme sans diabète sucré connu antérieurement.

La prééclampsie est une maladie caractérisée par l'association d'une hypertension artérielle, d'une protéinurie, d'une prise de poids avec oedèmes.

La menace d'accouchement prématuré est caractérisée par des contractions utérines. entraînant un raccourcissement et une ouverture du col pouvant provoquer un accouchement avant la fin de la 36$^{ème}$ semaine de gestation.

Par conséquent, un autre avantage de l'invention est de permettre le diagnostic et/ou le pronostic *in vitro,* ou le suivi *in vitro* de pathologies liées à des défauts de translocation du transporteur du glucose GLUT4 aussi bien dans les tissus que dans le sérum, le plasma ou les hématies.

Selon un autre mode de réalisation avantageux, l'utilisation du domaine extracellulaire de la protéine IRAP définie ci-dessus et/ou de l'une de ses isoformes définie ci-dessus, et/ou de l'un de ses variants défini ci-dessus, permet le diagnostic et/ou le pronostic *in vitro,* ou le suivi *in vitro* de pathologies liées à des défauts de translocation du transporteur du glucose GLUT4, notamment des cancers, en particulier ceux dans lesquels IRAP et/ou ses isoformes et/ou variants sont surexprimés tel que l'adénocarcinome ovarien, le cancer de l'endomètre, le choriocarcinome, le cancer du pancréas, le cancer du sein, le cancer de la prostate, le cancer de l'estomac, le cancer du rectum ou les cancers de la sphère ORL, les maladies auto-immunes ou inflammatoires.

Le choriocarcinome est une tumeur hautement maligne faite de la juxtaposition d'éléments cellulaires de cytotrophoblaste et de syncytiotrophoblaste avec disparition complète des villosités choriales.

Dans un mode de réalisation avantageux, la détection des cancers cités dans le document WO 2005/038462 par la méthode de l'invention est effectuée dans le sérum, mais également dans le plasma ou sur les hématies.

Par maladie auto-immune il faut comprendre des maladies dues à une hyperactivité du système immunitaire à l'encontre de substances ou de tissus qui sont normalement présents dans l'organisme, telles que les thyroïdites auto-immunes, la polyarthrite rhumatoïde, la spondylarthrite ankylosante, le syndrome de Goujerot-Sjögren.

Des exemples de maladies inflammatoires, mais sans être limités à ceux-ci, sont les suivants :

la polyarthrite rhumatoïde, le lupus érythémateux , syndrome de Sjögren, la sclérodermie (sclérose systémique), la dermatomyosite, la polymyosite, la polymyalgia rheumatica, l'ostéoarthrite, l'arthrite septique, la goutte, la pseudogoutte, la spondylarthropathies, la spondylite ankylosante, le syndrome de Reiter, l'arthropathie psoriatique, la spondylite entéropathique, l'arthropathie réactive, la maladie de Crohn, la sarcoïdose, ...

Dans le cadre d'un traitement par un médicament anti-cancéreux, IRAP réduit la sensibilité à ces médicaments en inhibant l'expression du facteur de promotion de l'apoptose et en augmentant l'expression du facteur inhibant l'apoptose (Kondo et al., Int. J. Cancer : 118, 1390-1394; 2006).

Selon un mode de réalisation plus avantageux, la mise en oeuvre d'une méthode de dosage *in vitro* au moyen du domaine extracellulaire de la protéine IRAP définie ci-dessus et/ou de l'une de ses isoformes définie ci-dessus, et/ou de l'un de ses variants défini ci-dessus est effectuée à l'aide d'un anticorps.

Le terme "anticorps" est utilisé pour désigner des anticorps polyclonaux ou monoclonaux spécifiques du domaine extracellulaire de l'une des isoformes de la protéine IRAP et comprend aussi des fragments ou des molécules qui miment les anticorps monoclonaux spécifiques du domaine extracellulaire de la protéine IRAP, et en particulier un fragment se liant à un épitope.

Des fragments ou des molécules peuvent être dérivés d'anticorps monoclonaux par des techniques d'ADN recombinant ou par des méthodes enzymatiques ou chimiques et peuvent présenter des caractéristiques de liaison similaires par rapport à un anticorps monoclonal pour un fragment d'antigène.

Les anticorps de la présente invention comprennent à la fois toute la longueur des anticorps discutés ci-dessus, ainsi que des fragments de ceux-ci se liant aux épitopes. Telle qu'elle est utilisé ci-après, l'expression « des fragments d'anticorps» comprend toute partie d'un anticorps qui conserve la capacité de se lier à un épitope reconnu par toute la longueur de l'anticorps, généralement appelé « fragments se liant à un épitope ».

Des exemples de fragments d'anticorps comprennent, mais ne se limitent pas à ceux-ci, Fab, Fab 'et F(ab')$_2$, Fd, simple chaîne Fvs (scFv), des anticorps simple chaîne, des Fvs liés par des ponts disulfure et des fragments comprenant soit une région VL ou VH. Des fragments se liant à un épitope, y compris des anticorps simple chaîne, peuvent comprendre la (les) région(s) variable(s) seule(s) ou en combinaison avec la totalité ou une partie des éléments suivants: région charnière, les domaines CH1, CH2, et CH3.

Ces fragments peuvent contenir un ou les deux fragments Fab ou le fragment F(ab')$_2$. En outre, les fragments peuvent être ou peuvent combiner les membres de l'une quelconque des catégories suivantes immunoglobulines: IgG, IgM, IgA, IgD, ou IgE, et les sous-classes de celle-ci.

Des fragments Fab et F(ab')$_2$ peuvent être produits par clivage protéolytique, en utilisant des enzymes telles que la papaïne (fragments Fab) ou la pepsine (fragments F(ab')$_2$).

Les anticorps de l'invention peuvent être produits à l'aide de méthodes conventionnelles, comprenant l'immunisation d'un animal et la récupération des cellules spléniques de manière à produire des hybridomes par fusion cellulaire.

Les anticorps de l'invention peuvent être utilisés avantageusement sous forme d'un mélange d'anticorps monoclonaux.

Selon un mode de réalisation avantageux, ledit anticorps défini ci-dessus est un anticorps polyclonal.

Par « anticorps polyclonal », on entend un anticorps provenant de différentes lignées de cellules de lymphocyte B.

Dans un mode de réalisation avantageux, ledit anticorps polyclonal défini ci-dessus est utilisé pour la mise en oeuvre d'une méthode de dosage *in vitro* de la concentration de la protéine IRAP définie ci-dessus et/ou de l'une de ses isoformes définie ci-dessus, et/ou variants dans du sérum, du plasma ou les hématies d'un mammifère, notamment d'un humain dans le cadre de l'insulino-résistance ou du cancer, ou dans des tissus d'un mammifère dans le cadre de l'insulino-résistance.

Selon un autre mode de réalisation avantageux, ledit anticorps défini ci-dessus est un anticorps monoclonal.

Par « anticorps monoclonal », on entend un anticorps provenant d'un seul clone de cellules, c'est-à-dire un hybridome.

Dans un mode de réalisation avantageux, ledit anticorps monoclonal défini ci-dessus est utilisé pour la mise en oeuvre d'une méthode de dosage *in vitro* de la concentration de la protéine IRAP définie ci-dessus et/ou de l'une de ses isoformes définie ci-dessus et/ou de l'un de ses variants, dans du sérum, du plasma, les hématies ou des tissus d'un mammifère, notamment de l'Homme dans le cadre de l'insulino-résistance ou du cancer.

Selon un mode de réalisation avantageux, l'anticorps monoclonal tel que défini ci-dessus est produit par un hybridome.

Par "hybridome" il faut entendre une cellule de fusion qui produit continuellement des anticorps, c'est-à-dire des cellules tumorales qui peuvent se reproduire sans fin et qui sont fusionnées avec des cellules de mammifères.

Selon un autre aspect, l'invention concerne un anticorps reconnaissant spécifiquement le domaine extracellulaire circulant, ou l'un des épitopes du domaine extracellulaire, de la protéine IRAP (insulin-responsive aminopeptidase) et/ou de l'une de ses isoformes, en particulier les isoformes définies par les SEQ ID N°1 à 3, et/ou de l'un de ses variants, en particulier les variants définis par les SEQ ID NO: 4 à 6, ledit domaine extracellulaire étant défini par les séquences SEQ ID NO : 7 à 11.

L'un des avantages de l'invention est donc de fournir un anticorps spécifique du domaine extracellulaire ou l'un des épitopes de la protéine IRAP et/ou de l'une de ses isoformes et/ou de l'un de ses variants, c'est-à-dire reconnaissant spécifiquement ledit domaine extracellulaire ou l'un des ses épitopes, que le domaine extracellulaire soit encore lié à la membrane plasmique de la cellule ou qu'il ait été clivé par des métalloprotéases appartenant ou non à la famille ADAM, notamment ADAM9 et ADAM12, et soit donc circulant et qui ne reconnaît pas la partie transmembranaire ou la partie intracellulaire.

Par conséquent, l'anticorps de l'invention reconnaît spécifiquement ledit domaine extracellulaire ou l'un des ses épitopes, après co-translocation avec le transporteur au glucose GLUT4, et permet donc une méthode de diagnostic et/ou de pronostic *in vitro,* ou de suivi *in vitro* de pathologies liées à des défauts de translocation du transporteur du glucose GLUT4 ou de protéines associées.

Dans un mode de réalisation avantageux, l'anticorps défini ci-dessus est utilisé en tant que médicament, notamment pour le traitement des syndromes prolifératifs et cancers, en particulier ceux dans lesquels IRAP et/ou ses isoformes et/ou variants sont surexprimés et/ou dont la translocation vers la membrane plasmique est accrue tel que l'adénocarcinome ovarien, le cancer de l'endomètre, le choriocarcinome, le cancer du pancréas, le cancer du sein, le cancer de la prostate, le cancer de l'estomac, le cancer du rectum ou les cancers de la sphère ORL, les maladies auto-immunes ou inflammatoires, ou pour le traitement de la résistance à la chimiothérapie.

La diminution de l'expression et/ou de sa translocation vers la membrane plasmique d'IRAP, c'est-à-dire, le retour à une concentration sérique ou dans les tissus proche de la normale, c'est-à-dire avant apparition de la pathologie cancéreuse ou l'inhibition partielle ou totale de son activité avec les anticorps de l'invention permet donc le traitement des cancers.

De même, la protéine IRAP étant impliquée dans la réduction de la sensibilité aux médicaments anticancéreux comme indiqué ci-dessus, les anticorps de l'invention permettent donc de réduire la concentration d'IRAP ou d'inhiber de façon partielle ou totale son activité et/ou sa translocation vers la membrane plasmique et donc de supprimer la réduction à la sensibilité aux médicaments anticancéreux conduisant au traitement de la résistance à la chimiothérapie et par voie de conséquence à l'amélioration de l'efficacité des anticancéreux.

Selon un mode de réalisation avantageux, l'anticorps défini ci-dessus est un anticorps polyclonal.

Selon un mode de réalisation avantageux, l'anticorps défini ci-dessus est un anticorps monoclonal.

Un autre mode de réalisation avantageux de l'invention concerne un anticorps monoclonal tel que défini ci dessus, le dit anticorps monoclonal étant choisi parmi :

- l'anticorps monoclonal sécrété par l'hybridome déposé à la CNCM (Collection Nationale de Culture de Microorganismes, Institut Pasteur, Paris, France) le 2 juillet 2009, sous le numéro d'accession CNCM I-4181,
- l'anticorps monoclonal sécrété par l'hybridome déposé à la CNCM le 2 juillet 2009, sous le numéro d'accession CNCM I-4182,

- l'anticorps monoclonal sécrété par l'hybridome déposé à la CNCM le 2 juillet 2009, sous le numéro d'accession CNCM I-4183,
- l'anticorps monoclonal sécrété par l'hybridome déposé à la CNCM le 2 juillet 2009, sous le numéro d'accession CNCM I-4184, et
- l'anticorps monoclonal sécrété par l'hybridome déposé à la CNCM le 2 juillet 2009, sous le numéro d'accession CNCM I-4185.

Dans ce qui suit, l'anticorps monoclonal n° CNCM I-4181 est également appelé anticorps 17H10-3H5-3D8, ou anticorps 17H10, l'anticorps monoclonal n° CNCM I-4182 est également appelé anticorps 14A4-3H9-2B6, ou anticorps 14A4, l'anticorps monoclonal n° CNCM I-4183 est également appelé anticorps 4G6-3B6, ou anticorps 4G6, l'anticorps monoclonal n° CNCM I-4184 est également appelé anticorps 38E1-2G4-3A2, ou anticorps 38E1, et l'anticorps monoclonal n° CNCM 1-4185 est également appelé anticorps 40C10-2G8, ou anticorps 40C10.

Selon un mode de réalisation avantageux, l'anticorps monoclonal est marqué avec un composé choisi parmi un radionucléide, un fluorophore, un quantum dot, un marqueur d'enzyme, un substrat d'enzyme, un co-facteur d'enzyme, un inhibiteur d'enzyme ou un haptène.

Le marqueur particulier ou le groupe détectable utilisé dans l'essai n'est généralement pas un aspect critique de l'invention, tant qu'il n'interfère pas avec la liaison spécifique de l'anticorps utilisés dans le dosage. Le groupe détectable peut être n'importe quel matériau ayant une propriété physique ou chimique détectable. De tels marqueurs détectables ont été bien développés dans le domaine des dosages immunologiques et, en général, presque tout marqueur utile dans de telles méthodes peut être appliqué à la méthode de la présente invention.

Ainsi, un marqueur est toute composition détectable par des techniques spectroscopiques, photochimiques biochimiques, immunochimiques, électriques, optiques, radiologiques ou des moyens chimiques. Des marqueurs utiles dans la présente invention incluent, mais ne se limitant pas à ceux-ci, sont des billes magnétiques (par exemple DynabeadsTM), des colorants fluorescents (par exemple, l'isothiocyanate de fluorescéine, texas rouge, la rhodamine), des marqueurs radiomarqués (par exemple, $^3$H, $^{125}$I, $^{35}$S, $^{14}$C, ou $^{32}$P), des enzymes (par exemple la peroxydase de raifort, de la phosphatase alcaline et d'autres couramment utilisés dans un test ELISA), et des marqueurs colorimétriques telles que l'or colloïdal, des billes de verre ou de plastique (par exemple le polystyrène, le polypropylène, latex, etc) colorées et les quantum dots.

Le marqueur peut être couplé directement ou indirectement à l'élément désiré de l'essai selon des méthodes bien connues dans l'art. Comme indiqué plus haut, une grande variété de marqueurs peut être utilisée, le choix du marqueur dépendant de la sensibilité nécessaire, la facilité de conjugaison avec le composé, des exigences de stabilité, les instruments disponibles et les conditions d'élimination. Des marqueurs radioactifs sont souvent attachés par des moyens indirects.

En règle générale, une molécule ligand (par exemple, la biotine) est liée de façon covalente à l'anticorps. Le ligand se lie alors à une molécule anti-ligand (par exemple streptavidine), qui est soit elle-même détectable soit liée de façon covalente à un système de signal, comme une enzyme détectable, un composé fluorescent, ou un composé chimioluminescent. Un certain nombre de ligands et d'anti-ligands peuvent être utilisés. Lorsqu'un ligand a un anti-ligand naturel, par exemple, la biotine, la thyroxine, et le cortisol, il peut être utilisé en association avec l'anti-ligand naturel marqué. Alternativement, un composé hapténique ou antigénique peut être utilisé en combinaison avec un anticorps.

Les anticorps peuvent aussi être conjugués directement à des composés générant un signal, par exemple, en conjugaison avec une enzyme ou un fluorophore. Les enzymes d'intérêt, utilisés comme marqueurs sont principalement des hydrolases, en particulier des phosphatases, des estérases et des glycosidases, ou des oxidoréductases, en particulier des peroxidases.

Des composés fluorescents comprennent la fluorescéine et ses dérivés, la rhodamine et ses dérivés, le dansyl, l'umbelliferone, etc. Des composés chimioluminescent comprennent la luciférine, et les 2,3-dihydrophtalazinediones; par exemple, le luminol et les quantum dots. Une revue des marqueurs ou d'autres systèmes de production du signal est disponible dans le brevet américain n° 4,391,904.

Des moyens pour détecter les marqueurs sont bien connus dans l'art. Ainsi, par exemple, quand le marqueur est un marqueur radioactif, les moyens de détection comprennent un compteur γ ou β à scintillation ou des films photographiques comme pour l'autoradiographie. Lorsque le marqueur est un marqueur fluorescent, il peut être détecté par excitation du fluorophore aux longueurs d'onde de la lumière ou laser appropriés et détection de la fluorescence résultante. La fluorescence peut être détecté visuellement, par le biais d'un film photographique, par l'utilisation de détecteurs électroniques tels que les dispositifs à couplage de charge (CCD) ou des photomultiplicateurs et les équivalents.

De même, des marqueurs enzymatiques peuvent être détectés en fournissant les substrats appropriés à l'enzyme et en détectant le produit de réaction-résultant. Enfin de simples marqueurs colorimétriques peuvent être détectés simplement en observant la couleur associée au marqueur.

Selon un mode de réalisation avantageux, ledit anticorps monoclonal défini ci-dessus est un anticorps humanisé.

Par « anticorps humanisé » il faut entendre un anticorps génétiquement modifié dans lequel la partie minimum d'un anticorps de souris est intégrée à un anticorps humain; généralement les anticorps humanisés comprennent de 5-10% d'anticorps de souris et de 90 à 95% d'anticorps humain.

Les anticorps humanisés ont l'avantage de bloquer les réponses HAMA (anticorps humains dirigés contre des anticorps de souris) et HACA (anticorps humains dirigés contre des anticorps chimériques) observées avec l'utilisation d'anticorps de souris ou chimériques et qui ne présentent seulement qu'une réponse minimale ou pas de réponse du système immun humain contre eux.

Selon un autre aspect, l'invention concerne un hybridome produisant un anticorps monoclonal tel que défini ci-dessus.

Un autre aspect avantageux de l'invention concerne un hybridome tel que défini précédemment, ledit hybridome étant chois parmi :

- l'hybridome déposé à la CNCM le 2 juillet 2009, sous le numéro d'accession CNCM I-4181,
- l'hybridome déposé à la. CNCM le 2 juillet 2009, sous le numéro d'accession CNCM I-4182,
- l'hybridome déposé à la CNCM le 2 juillet 2009, sous le numéro d'accession CNCM I-4183,
- l'hybridome déposé à la CNCM le 2 juillet 2009, sous le numéro d'accession CNCM 1-4184, et
- l'hybridome déposé à la CNCM le 2 juillet 2009, sous le numéro d'accession CNCM I-4185.

L'obtention de ces hybridomes est décrite dans les Exemples.

Selon un autre aspect, l'invention concerne un procédé de dosage *in vitro* de la concentration en protéine IRAP, et/ou de l'une de ses isoformes, et/ou de l'un de ses variants chez un mammifère comprenant une étape de détermination de la concentration du domaine extracellulaire de la protéine IRAP et/ou de l'une de ses isoformes et/ou de l'un de ses variants dans du sérum, du plasma, les hématies ou des tissus d'un mammifère.

Le procédé de l'invention permet donc le dosage spécifique du domaine extracellulaire de la protéine IRAP après co-translocation avec le transporteur du glucose GLUT4 et permet donc de déterminer la surexpression ou la sous-expression et/ou l'augmentation ou la diminution de la translocation de la protéine IRAP et/ou de l'une de ses isoformes et/ou de l'un de ses variants responsable de pathologies associées.

Procédé de dosage selon la revendication 18, dans lequel le dosage est effectué soit par une méthode immuno-enzymatique soit par une méthode immuno-histochimique, soit par RIA ou IRMA.

Le dosage du domaine extracellulaire de la protéine IRAP peut être effectué soit par dosage immuno-enzymatique (exemple 3), soit par dosage immuno-histochimique (exemple 4), RIA (radio immuno-assay) ou IRMA (immunoradiometric assay). Dans ces derniers cas, le dorage est effectué à l'aide, soit d'anticorps, soit de la protéine ou d'un fragment de celle-ci, marqués à l'iode 125 ou avec tout autre radioisotope approprié.

Selon un autre aspect, l'invention concerne une méthode de diagnostic *in vitro* de pathologies associées à l'insulino-résistance ainsi que le diabète de type 2, le diabète gestationnel, l'hypertension gravidique (prééclampsie), la menace d'accouchement prématuré et comprenant les étapes suivantes :

a. la détermination *in vitro* de la concentration de domaine extracellulaire circulant de la protéine IRAP, c'est à dire aussi exprimé au niveau de la membrane plasmique des hématies, et/ou de l'une de ses isoformes, et/ou de l'un de ses variants, chez un mammifère à l'aide d'un anticorps tel que défini ci-dessus,
b. la comparaison de ladite concentration obtenue à l'étape a. avec celle obtenue in *vitro* chez un mammifère sain,
c. la déduction à partir de l'étape b. précédente, du fait que le mammifère présente une insulino-résistance, si la concentration obtenue à l'étape a. est inférieure à celle de l'étape b.,

Un autre avantage de l'invention est donc de fournir une méthode diagnostique et/ou le pronostique *in vitro,* ou le suivi *in vitro* de pathologies dans lesquelles la protéine IRAP et/ou l'une de ses isoformes et/ou de l'un de ses variants est sous-exprimée.

Selon un autre aspect, la présente invention concerne une méthode de diagnostic *in vitro* de pathologies associées aux syndromes prolifératifs y compris les cancers, en particulier ceux dans lesquels IRAP, et/ou l'une de ses isoformes et/ou l'un de ses variants, est surexprimée et/ou dont la translocation vers la membrane plasmique est augmentée tels que l'adénocarcinome ovarien, ou à des maladies auto immunes ou inflammatoires, ou à la résistance à la chimiothérapie et comprenant les étapes suivantes :

a. la détermination *in vitro* de la concentration de domaine extracellulaire circulant de la protéine IRAP c'est à dire aussi exprimé au niveau de la membrane plasmique des hématies, et/ou de l'une de ses isoformes, et/ou de l'un de ses variants, chez un mammifère à l'aide d'un anticorps tel que défini ci-dessus,
b. la comparaison de ladite concentration obtenue à l'étape a. avec celle obtenue *in vitro* chez un mammifère contrôle,

EP 2 297 205 B1

c. la déduction à partir de l'étape b. précédente, du fait que le mammifère présente un cancer, si la concentration obtenue à l'étape a. est supérieure à celle de l'étape b.

Un autre avantage de l'invention est donc de fournir une méthode diagnostique et/ou pronostique *in vitro,* ou le suivi *in vitro* de pathologies dans lesquelles la protéine IRAP, et/ou l'une de ses isoformes, et/ou de l'un de ses variants, est surexprimée ou dont la translocation à la membrane plasmique est augmentée.

Selon encore un autre aspect, la présente invention concerne un kit pour la détermination *in vitro* de la concentration de domaine extracellulaire circulant de la protéine IRAP, et/ou de l'une de ses isoformes et/ou variants, chez un mammifère comprenant au moins un tampon, et au moins un anticorps tel que défini ci-dessus.

Dans un mode de réalisation préféré, le kit défini ci-dessus, comprend également un substrat de la protéine IRAP ainsi qu'un peptide ou un fragment d'IRAP reconnu par l'anticorps.

Des exemples de substrat, sans être limités à ceux-ci sont la L-leucine-nitroanilide, la vasopressine, l'ocytocine, les met-enkephalines....

Par « peptide reconnu par l'anticorps » il faut comprendre la protéine IRAP recombinante ou un fragment de celle-ci reconnu par l'anticorps.

Selon un autre aspect, la présente invention concerne une méthode de diagnostic *in vitro* de pathologies associées aux cancers, en particulier ceux dans lesquels IRAP, et/ou l'une de ses isoformes et/ou l'un de ses variants, est surexprimée ou dont la translocation à la membrane plasmique est augmentée tel que l'adénocarcinome ovarien, ou à des maladies auto immunes ou inflammatoires, ou à la résistance à la chimiothérapie et comprenant les étapes suivantes :

a. la détermination *in vitro* de la concentration de domaine extracellulaire circulant de la protéine IRAP, et/ou de l'une de ses isoformes, et/ou de l'un de ses variants, chez un mammifère à l'aide d'au moins un anticorps monoclonal tel que défini ci-dessus, préférentiellement 2 anticorps monoclonaux tels que définis ci-dessus, notamment les anticorps 17H10 et 4G6 ou 40C10,
b. la comparaison de ladite concentration obtenue à l'étape a. avec celle obtenue *in vitro* chez un mammifère contrôle,
c. la déduction à partir de l'étape b. précédente, du fait que le mammifère présente un cancer, si la concentration obtenue à l'étape a. est supérieure à celle de l'étape b.

Selon un autre aspect, l'invention concerne une méthode de diagnostic *in vitro* de pathologies associées à l'insulino-résistance ainsi que le diabète de type 2, le diabète gestationnel, l'hypertension gravidique (prééclampsie), la menace d'accouchement prématuré et comprenant les étapes suivantes :

a. la détermination *in vitro* de la concentration de domaine extracellulaire circulant de la protéine IRAP, et/ou de l'une de ses isoformes, et/ou de l'un de ses variants, chez un mammifère à l'aide à l'aide d'au moins un anticorps monoclonal tel que défini ci-dessus, préférentiellement 2 anticorps monoclonaux tels que définis ci-dessus, notamment les anticorps 17H10 et 4G6 ou 40C10,
b. la comparaison de ladite concentration obtenue à l'étape a. avec celle obtenue *in vitro* chez un mammifère sain,
c. la déduction à partir de l'étape b. précédente, du fait que le mammifère présente une insulino-résistance, si la concentration obtenue à l'étape a. est inférieure à celle de l'étape b.

Les figures et les exemples suivants illustreront mieux l'invention, sans pour autant en limiter sa portée.

Les figures 1A-E représentent des graphiques correspondant à la mesure de l'affinité des anticorps monoclonaux 17H10, 14A4, 4G6, 38E1, 40C10 pour la protéine IRAP.

La figure 1A montre un graphique mesurant la détection de la protéine IRAP (■) ou IRAP His (♦) en fonction de la concentration de l'anticorps 4G6.

La figure 1B montre un graphique mesurant la détection de la protéine IRAP (▲) ou IRAP His (X) en fonction de la concentration de l'anticorps 14H4.

La figure 1C montre un graphique mesurant la détection de la protéine IRAP (●) ou IRAP His (*) en fonction de la concentration de l'anticorps 17H10.

La figure 1D montre un graphique mesurant la détection de la protéine IRAP (+) ou IRAP His (-) en fonction de la concentration de l'anticorps 38E1.

La figure 1E montre un graphique mesurant la détection de la protéine IRAP (♦) ou IRAP His (--) en fonction de la concentration de l'anticorps 40C10.

La figure 2 représente la courbe dose réponse de détection d'IRAP dans un ELISA où l'anticorps monoclonal 17H10 est utilisé en tant qu'anticorps de capture et l'anticorps monoclonal 4G6 en tant qu'anticorps de détection. (♦) représente le dosage d'IRAP recombinant, (■) représente le dosage d'IRAP dans le sérum de femme enceinte, et (▲) représente le dosage d'IRAP dans le sérum d'homme.

La figure 3 représente la courbe dose de saturation de la détection d'IRAP dans un ELISA où l'anticorps monoclonal 17H10 est utilisé en tant qu'anticorps de capture et l'anticorps monoclonal 40C10 en tant qu'anticorps de détection, (♦) représente le dosage d'IRAP recombinant, (■) représente le dosage d'IRAP recombinant dilué dans du sérum de femme enceinte, et (▲) représente le dosage d'IRAP recombinant dilué dans du sérum d'homme.

## EXEMPLES

### EXEMPLE 1 : Production de la protéine IRAP

1° clonage:

Un vecteur d'expression contenant la séquence codant du domaine extracellulaire d'IRAP en fusion avec un peptide signal d'insecte et une étiquette 6 histidines clivable à laTev protease en N-terminal a été construit et sa conformité avec la séquence de la base de données Swiss-Prot (Q9UIQ6) a été vérifiée par séquençage (pGTPb302-mel-His-Tev-PLAPextra,référence C640-CAP-06).

Un bacmide recombinant a été élaboré et la présence de la cassette d'expression dans le bacmide généré a été contrôlée par PCR.

Le virus recombinant a été généré par transfection du bacmide dans la lignée cellulaire d'insectes *Sf*9.

Après une amplification virale, le virus de deuxième génération a été titré et utilisé pour une série d'essais d'expression en système cellules d'insectes/baculovirus.

2° Expression et purification de la séquence extracellulaire circulante d'IRAP ou de l'une de ses isoformes ou de l'un de ses variants. Trois méthodes différentes peuvent être utilisées :

- 1° méthode : expression de la protéine IRAP ou de l'une de ses isoformes par une souche appropriée d'E. coli. La protéine IRAP ou l'une de ses isoformes sécrétée est ensuite purifiée par affinité sur matrice de $Ni^{++}$ ou $Co^{++}$. Enfin, les séquences poly-His sont clivées à l'aide d'une protéase spécifique.

- 2° méthode expression de la protéine IRAP ou de l'une de ses isoformes ou de l'un de ses variants par des cellules Sf9 d'insecte après clonage du cDNA modifié dans Baculovirus. : Les essais d'expression ont été réalisés sur 2 lignées cellulaires : Sf9 et HighFive en faisant varier le MOI (multiplicity of infection) ainsi que la durée d'infection. L'analyse des essais d'expression a été réalisée par Western Blot anti His sur des échantillons de surnageant de culture prélevés à 24, 48 et 72 heures d'infection et la condition la plus productive est à 48 heures d'infection (HighFive MOI 0,1)

La protéine IRAP ou l'une de ses isoformes ou de l'un de ses variants sécrétée est ensuite purifiée par affinité sur matrice de $Ni^{++}$ ou $Co^{++}$. Enfin, les séquences poly-His sont clivées à l'aide d'une protéase spécifique.

### EXEMPLE 2 : Production des anticorps monoclaux

GENERALITE

Le protocole est issu de la technique de l'hybridome (protocole de Kôhler et Milstein, 1976) et se divise en 5 étapes :

- **Etape 1 - Immunisation :** injection sous-cutanée à des souris de plusieurs peptides correspondant à des séquences spécifiques communes de la partie extracellulaire des isoformes d'IRAP ou de l'un de ses variants. Ces séquences peptidiques ne sont pas retrouvées dans les autres aminopeptidases de mammifères.

Les antisérums sont testés contre la protéine recombinante purifiée IRAP ou l'une de ses isoformes par ELISA. Les rates des souris répondantes sont utilisées pour la génération d'hybridomes. Les anticorps produits par ceux-ci sont ensuite retestés par ELISA contre la protéine IRAP ou de l'une de ses isoformes et du sérum de femmes enceintes et non gravides.

- **Etape 2 - Fusion** : prélèvement des cellules de rates et fusion de ces cellules avec des cellules de myélome en présence de polyéthylène glycol (agent chimique de fusion). Distribution du mélange dans des puits de microplaque à une dilution telle qu'en moyenne chaque puits contient moins d'une cellule hybride. Culture des cellules dans un milieu sélectif où seules les cellules hybrides se multiplient et perdurent, tandis que les cellules myélomateuses et les plasmocytes de rate non fusionnés meurent rapidement.

- **Étape 3 - Criblage : recherche** dans chaque puits d'anticorps dirigés contre la protéine IRAP ou de l'une de ses isoformes ou de l'un de ses variants recombinante.

- **Etape 4 - Clonage et caractérisation :** repiquage des cellules hydrides produisant des anticorps afin d'obtenir des clones cellulaires. Conservation d'une copie de chaque clone dans l'azote liquide. Isotypage des anticorps produits.

- **Etape 5 - Culture et production :** deux modes possibles de culture des hybridomes pour produire les anticorps anti-IRAP ou de l'une de ses isoformes ou de l'un de ses variants:

  ○ culture in vitro des cellules (production des anticorps dans le milieu de culture)
  ○ culture in vivo par injection des cellules dans la cavité péritonéale de souris. Ceci provoque l'apparition d'une tumeur, d'une inflammation et d'une production de liquide d'ascite dans la zone d'injection. Prélèvement du liquide d'ascite qui contient les anticorps (1 à 10 mg/mL).

HYBRIDOMES SPECIFIQUES

**1- Immunisation**

6 souris femelles OF1 Charles River (18-20g) ont été immunisées par injection intraveineuse et sous-cutanée d'un mélange de peptides SEQ ID NO 7 à 11, les 5 peptides étant couplés à la KLH (hémocyanine de Megathura crenulata) en présence d'adjuvant complet de Freund. 3 souris (souris 1 à 3) ont reçu 50$\mu$g du mélange des peptides et 2 souris (souris 4 et 5) ont reçu 15 $\mu$g du mélange des peptides.

3 semaines après la première injection, les souris ont été réinjectées avec le mélange des deux peptides en présence d'adjuvant incomplet de Freund (1$^{er}$ rappel).

3 semaines après la seconde injection, les souris ont été réinjectées avec le mélange des deux peptides en présence d'adjuvant incomplet de Freund (deuxième rappel).

1 mois après la première injection, des échantillons de sérum des souris injectées a été prélevé, et lesdits sérums ont été testés pour la présence d'anticorps dirigés contre les peptides SEQ ID NO 7 à 11.

Les sérum de toutes les souris présentaient des anticorps reconnaissant au moins un des peptides et ont ainsi été conservés.

10 jours après le test du sérum, 3 souris ont reçu un boost intra-péritonéal et intraveineux de 20 $\mu$g du mélange des peptides. Les rates ont été prélevées 3 jours après le boost.

1 mois après le test du sérum, les 3 souris restantes ont reçu un boost intra-péritonéal et intraveineux de 15 $\mu$g du mélange des peptides. Les rates ont été prélevées 3 jours après le boost.

**2- Fusion cellulaire**

Les souris ont été saignées et leur rate a été prélevée stérilement avec du DMEM. Les rates ont été broyées et filtrées sur grille.

En parallèle, la cavité intraperitonéale des souris est lavée avec du DMEM, et le DMEM comprenant des macrophages est récupérée. Les macrophages ont été compté dans une cellule de Malassez afin de préparer une solution à 104 macrophages / ml en milieu DMEM HAT SVF 20% ATB (DMEM, 4mM glutamine, HAT (hypoxantine 100$\mu$M, aminoptérine 0,4 $\mu$M, thymidine 16 $\mu$M), 20% sérum de veau foetal décomplémenté, 1% antibiotiques (Pénicilline/Streptomycine)).

Les splénocytes obtenus ont alors été lavés trois fois avec du DMEM

En parallèle, des cellules de myélome de souris BalB/c Sp2/O Ag14 (ATCC n° CRL 1581) sont également lavées 3 fois dans du DMEM

Les splénocytes et les cellules de myélome ont été mélangées avec un rapport splénocytes/myélome de 5/1 et centrifuger à 244g pendant 7 minutes.

Le surnageant a été éliminé et 1 ml de PEG (solution à 40% de polyéthylène glycol, PM 1500 chauffée à 37°C) a été ajoutée.

Les cellules ont été centrifugées à 800 rpm (108 g) pendant 12 minutes, 10 ml de milieu DMEM HAT SVF 20% (DMEM, 4mM glutamine, HAT (hypoxantine 100$\mu$M, aminoptérine 0,4 $\mu$M, thymidine 16 $\mu$M), 20% sérum de veau foetal décomplémenté) a été ajouté lentement.

Les cellules ont été centrifugées à 1200 rpm (244 g) pendant 7 minutes, le surnageant a été éliminé et sur le culot a été ajouté un volume v de milieu DMEM HAT SVF 20% tel que :

$$v \text{ (en ml) = nb de splénocytes / } 10^7 \text{ (soit } 10^7 \text{ cellules/ml)}$$

Le tube a été laissé à température ambiante pendant 1 heure avant d'être retourné délicatement pour remettre les cellules en suspension.

Dans des plaques de 96 puits, 100 $\mu$l/puits de la solution à 104 macrophages / ml a été ajoutée puis 100$\mu$l par puits de cellules fusionnées ont été ajoutés aux dilutions suivantes :

dilution 1/10 : 3 plaques à 105 splénocytes par puits
dilution 1/20 : 5 plaques (2x 50ml) à 5 x104 splénocytes par puits
dilution 1/40 : 2 plaques à 2,5 x104 splénocytes par puits

Les plaques ont été placées à l'étuve à 37°C, 5%CO2 pendant 10 jours.

### 3- Sélection des hybridomes

Après 10 jours de culture des produits de fusion deux tests de sélection sont réalisés :

- Test 1 : Les puits dont les cellules sont arrivées à confluence sont analysés :

 - pour les fusions issues des splénocytes des 3 premières souris , 1017 puits ont été analysés,
 - pour les fusions issues des splénocytes des 3 dernières souris, 714 puits ont été analysés.

 100 $\mu$l de surnageant de chacun de ces puits a été prélevé et les surnageants ont été testés par un test ELISA pour la détection des anticorps dirigés contre un ou plusieurs peptides SEQ ID NO 7 à 11 (cf. Criblage des surnageants des hybridomes anti-IRAP).
 Après le test ELISA, les cellules sélectionnées (sécrétant des anticorps dirigés contre un ou plusieurs peptides SEQ ID NO 7 à 11) ont été passées dans 0,4 ml de milieu en puits d'une plaque de 24 puits.
 Lorsque les cellules commençaient à se multiplier (24 à 48 heures) à 1ml du milieu DMEM HAT SVF 15% HCF 1% ATB (DMEM, 4mM glutamine, HAT (hypoxantine 100$\mu$M, aminoptérine 0,4 $\mu$M, thymidine 16 $\mu$M), 15% sérum de veau foetal décomplémenté, 1% HCF (hybridoma cloning factor macrophage-like origin), 1% antibiotiques (Pénicilline/Streptomycine)) a été ajouté.
 111 clones ont ainsi été sélectionés et congelés

- Test 2 : Les puits dont les cellules issues des 111 clones sélectionés par le test 1, sont arrivées à confluence sont analysés :

 - pour les fusions issues des splénocytes des 3 premières souris, 39 puits de plaque 24 puits ont été analysés,
 - pour les fusions issues des splénocytes des 3 dernières souris, 72 puits de plaque 24 puits ont été analysés.

 100 $\mu$l de surnageant de chacun de ces puits a été prélevé et les surnageants ont été testés par un test ELISA pour la détection d'anticorps dirigés contre le domaine sécrété d'IRAP (cf. Criblage des surnageants des hybridomes anti-IRAP).
 23 clones ont ainsi été sélectionnés et congelés.

### 4- Criblage des surnageants des hybridomes anti-IRAP

Antigènes (Ag) utilisés: KLH-peptides SEQ ID NO 7 à 11 (1er screening) et domaine sécrété d'IRAP recombinant exprimé dans des cellules d'insecte High Five (second screening).

| ETAPES | CONDITIONS |
|---|---|
| Coating (adsorption de l'Ag.) | Plaque 96puits (Maxisorp, Nunc) |
| Concentration de l'Ag | 1 $\mu$g/ml |
| Tampon | PBS |

(suite)

| ETAPES | CONDITIONS |
|---|---|
| Volume / puits<br>Incubation | 50 $\mu$l<br>1 nuit à température ambiante |
| Lavage : x1 | PBS - 0,05% (v/v) Tween 20 |
| **Saturation**<br>Tampon<br>Volume / puits<br>Incubation | PBS-lait 2,5% (p/v)<br>150 $\mu$l<br>1h à 25°C |
| Lavage: x1 | PBS - 0,05% (v/v) Tween 20 |
| **Anticorps à tester**<br>Surnageant de culture pur<br>Volume / puits<br>Incubation | 50 $\mu$l<br>2h à 25°C |
| Lavage : x3 | PBS - 0,05% (v/v) Tween 20 |
| **Anticorps secondaire**<br>(conjugué peroxydase)<br>Dilution<br>Tampon<br>Volume /puits<br>Incubation | Anti-IgG et IgM (115-036-044, Jackson)<br>1/10 000<br>PBS-0,05% (v/v) Tween 20-0,5% (p/v) BSA<br>50 $\mu$l<br>1h à 25°C |
| Lavage : x3 | PBS - 0,05% (v/v) Tween 20 |
| **Révélation**<br>Réactif<br>Volume /puits<br>Incubation | Tetramethylbenzidine (50-76-05, KPL, Inc.)<br>50 $\mu$l<br>10 min |
| **Arrêt de la réaction**<br>**Volume** | $H_2SO_4$ 1M (S1526, Sigma) 50 $\mu$l |

### 5- Isotypage des anticorps

L'isotype des anticorps a été déterminé en utilisant le kit SouthernBiotech SBA Clonotyping System/HRP (Cliniscience, Montrouge, France) de la façon suivante :

### 1. Coating des plaques

Diluer l'anticorps anti-immunoglobulines de souris à la concentration de 5 $\mu$g/ml. Déposer 50 $\mu$l par puits et incuber 1 heure à 37°C ou 16 heures à température ambiante.

### 2. Lavage

Rincer 1 fois avec 200$\mu$l/puit de PBS-Tween20 0,05%%(v/v).

### 3. Blocage

Ajouter dans chaque puits 150 $\mu$l de PBS-Lait 2,5%(p/v) et incuber 1 heure à 37°C.

### 4. Lavage

Rincer 1 fois avec le tampon PBS-Tween20 0,05%(v/v).

### 5. Préparation des échantillons d'anticorps à tester

Diluer les surnageants de culture de l'hybridome au 1/10 en PBS-Tween20 0,05%(v/v)-BSA 0,5%(p/v). Déposer 50 μl par puits et incuber 2 heures à température ambiante.

### 6. Lavage

Rincer 3 fois avec le tampon PBS-Tween20 0,05%(v/v).

### 7. Anticorps secondaire

Déposer 50 μl par puits d'anticorps anti-souris IgA, IgG1, IgG2a, IgG2b, IgG3 ou IgM conjugués à la peroxidase (HRP) (dilués au 1/2000 en PBS-Tween20 0,05%(v/v)-BSA 0,5%(p/v)) et incuber 1 heure à température ambiante.

### 8. Lavage

Rincer 3 fois avec le tampon PBS-Tween20 0,05%(v/v).

### 9. Réaction avec le substrat

Déposer 50 μl par puits de Tetramethylbenzidine (KPL, Inc.) et incuber la plaque 10 minutes à température ambiante.

### 10. Arrêt de la réaction

Ajouter 50 μl de $H_2SO_4$ dans chaque puits et lire l'absorbance à 450 nm, au lecteur de microplaques (Dynex).

### 6- Résultats

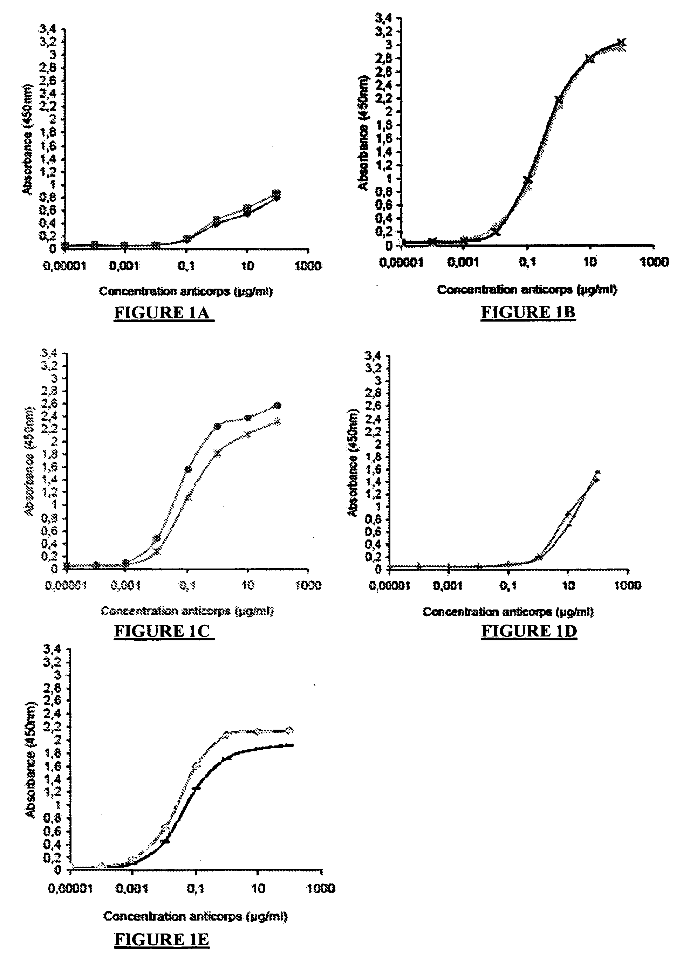

5 hybridomes ont été conservés: 17H10, 14A4, 4G6, 38E1, 40C10 pour leur excellente affinité pour un des peptides SEQ ID NO 7 à 11, ainsi que pour le domaine sécrété d'IRAP recombinant comme indiqué dans les figures 1A-.E
Chacun des anticorps 17H10, 14A4, 4G6, 38E1, 40C10 a été testé par ELISA sur des plaques dans lesquelles 1 mg/mL d'IRAP, ou 2mg/mL d'IRAP étiqueté avec une étiquette 6 His, a été immobilisé. Les anticorps sont incubés dans les plaques, et leur détection est révélée à l'aide d'un anticorps secondaire couplés à la peroxidase (HRP) reconnaissant la partie constante des anticorps monoclonaux. Le complexe immun IRAP-anticorps monoclonal-anticorps marqué est révélé du TMB (3,3',5,5'-tetramethylbenzidine), un substrat chromogène de la peroxidase dont la couleur vire au bleu en présence de peroxyde d'hydrogène et dont la couleur devient jaune en présence d'acide sulfurique (arrêt de la réaction). La réaction peut être quantifiée par détection à 450 nm.
Les résultats obtenus montrent que les anticorps monoclonaux sélectionnés peuvent être utilisés comme anticorps pour la détection du domaine sécrété d'IRAP en ELISA sandwich. La courbe dose - réponse indique une limite de détection du domaine sécrété d'IRAP de 1 à 100 ng/ml selon l'anticorps.
Les clones ont été conservés et clonés en réalisant des dilutions limites dans des plaques 96 puits contenant 10, 5, 3, 1 ou 5 cellules par puits en moyenne.
Les produits du clonage ont été congelés dans du milieu DMEM HT SVF 15% HCF 1% ATB (2) : DMEM, 4mM glutamine, HAT (hypoxantine 100μM, thymidine 16 μm), 15% sérum de veau foetal décomplémenté, 1% HEF (hybridoma enhancing supplement), 1% antibiotiques (Pénicilline/Streptomycine) complété avec 10% DMSO (Diméthylsulfoxide)

### EXEMPLE 3 : Dosage de la protéine IRAP et/ou de l'une de ses isoformes et/ou de l'un de ses variants par une méthode immuno-enzymatique dans le sérum ou le plasma

La protéine IRAP et/ou l'une de ses isoformes recherchée dans le sérum ou le plasma est liée par l'anticorps spécifique adsorbé à des plaques multi-puits. La protéine IRAP ainsi que ses isoformes, voir l'un ou plusieurs de ses variants liés sont ensuite révélés et quantifiés par mesure de leur activité enzymatique en utilisant le L-leucine-paranitroanilide comme substrat en présence de 20 mM de L-methionine, cette dernière permettant d'éviter une réaction croisée avec d'éventuelles contaminations par d'autres aminopeptidases (Yamahara, N., Nomura, S., Suzuki, T., Itakura, A., Ito, M., Okamoto, T., Tsujimôto, M., Nakazato, H., and Mizutani, S. (2000) Life Sci 66(15), 1401-1410).
Les méthodes enzymatiques, et notamment utilisant le substrat L-leucine-paranitroanilide permettent de détecter IRAP à partir d'une concentration de 10μg/mL. Un enrichissement d'IRAP par immunocapture réduit le seuil de détection à

1μg/mL.

Cependant ces seuil de détection élevés sont peu compatible avec la détection d'IRAP dans le sérum,

Aussi, l'utilisation d'anticorps monoclonaux très spécifiques pour purifier IRAP est indispensable, afin de diminuer le seuil de détection.

Afin de valider l'efficacité des anticorps monoclonaux, un ELISA sandwich a été réalisé en utilisant les anticorps 17H10 et 4G6.

L'anticorps monoclonal 17H10 est utilisé en tant qu'anticorps de capture à 15 μg/ml et l'anticorps monoclonal 4G6 est utilisé en tant qu'anticorps de détection. Les complexes c 17H10-IRAP-4G6 sont révélés par un anticorps anti-isotype dirigé contre les IgG2a de souris conjugué à la peroxydase (HRP), à partir d'échantillons d'IRAP recombinant, ou de plasma de femme enceinte (14-16 semaines) ou d'homme.

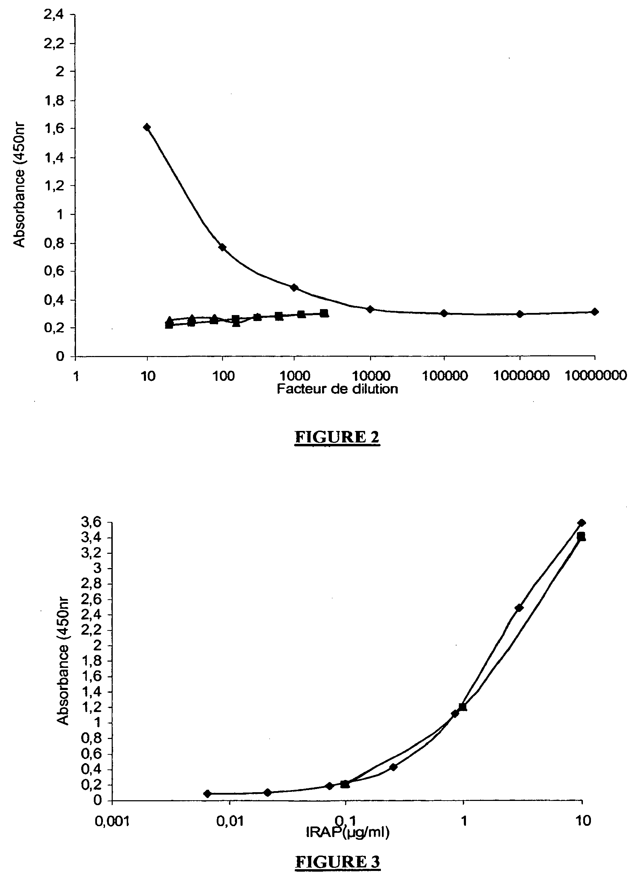

Les résultats sont indiqués dans la figure 2.

Dans ces conditions d'utilisation, la combinaison d'anticorps 17H10 - 4G6 -anti-IgG2a/HRP permet de détecter le domaine sécrété de la protéine IRAP jusqu'à une concentration de 0,1 μg/ml.

Cette combinaison ne permet pas de détecter IRAP dans ces plasmas aux dilutions testées. L'utilisation d'un quatrième anticorps anti-HRP conjugué à la phosphatase alcaline permet d'augmenter la sensibilité jusqu'à 1 ng/ml.

**EXEMPLE 4 : Dosage de la protéine IRAP et/ou de l'une de ses isoformes et/ou de l'un**

**de ses variants par une méthode immuno-histochimique dans les tissus humains.**

**Le** dosage immunohistochimique a été effectué en utilisant la technique avidine-biotine immunoperoxydase. Des coupes de tissus humains préalablement prélevés d'une épaisseur de 4 μm ont été effectuées et marquées par la méthode streptavidine/biotine/peroxydase.

Les coupes débarrassées de leur paraffine ont été placées dans un tampon citraté 0.01 M et été traitée trois fois pendant 5 min chacune à 90°C et 750 W dans un four à micro-ondes.

Les coupes ont été ensuite incubées dans de l'eau oxygénée pendant 20 min et puis incubées avec du sérum de l'animal hôte de l'anticorps secondaire à 10% pendant 10 min pour bloquer l'activité peroxydase endogène et la liaison aux immunoglobulines non spécifiques, respectivement.

Un anticorps monoclonal de l'exemple 2 à une dilution de 1: 100 a été ajouté aux coupes de tissus et incubé pendant 1h dans une chambre humide à température ambiante pour le dosage de IRAP et/ou de ses isoformes et/ou de l'un de ses variants.

La liaison de l'anticorps a été révélée par un anticorps anti-Ig de souris biotinylé, suivi de streptavidine conjuguée à la peroxydase de raifort.

Le développement chromogénique a été effectué par immersion des coupes dans du 3-amino-9-ethylcarbazole.

Les clichés ont été analysés par coloration de contraste avec de l'hématoxyline de Mayer.

**EXEMPLE 5 : Dosage de la protéine IRAP et/ou de l'une de ses isoformes et/ou de l'un de ses variants pour le pronostic de la chimiorésistance.**

La chimiorésistance est la conséquence de la surexpression d'IRAP. Elle peut donc être dosée selon l'exemple 3 ou 4 ou par RIA ou IRMA.

**EXEMPLE 6 : Dosage de la protéine IRAP et/ou de l'une de ses isoformes et/ou de l'un de ses variants pour mesurer la survenue de l'accouchement précoce.**

**La concentration d'IRAP augmente au cours de la grossesse.** Il peut donc être prédit en cas de baisse de la concentration circulante d'IRAP dosée selon l'exemple 3 ou 4 ou par RIA ou IRMA.

**EXEMPLE 7 : Spécificité des anticorps monoclonaux dirigés contre la partie extracellulaire d'IRAP.**

Afin de valider la spécificité des anticorps de l'invention, des mesures d'interaction IRAP-anticorps ont été réalisées en présence de sérums dilués ou non.

L'interaction anticorps-IRAP a été mesurée en fonction de la concentration en IRAP recombinant, ou d'IRAP recombinant dilué dans du sérum d'homme ou de femme enceinte dilué au 1/100e, à l'aide d'un ELISA sandwich utilisant les anticorps 17H10 et 40C10.

Les résultats sont montrés dans la figure 3.

Ces résultats montrent que IRAP est détectable à une concentration de 0,1μg/mL dans du sérum dilué, sans qu'il y ait d'interférence avec les autres aminopeptidases contenues dans le sérum.

Ces résultats montrent que les anticorps monoclonaux sont très spécifiques d'IRAP.

## Revendications

1. Utilisation du domaine extracellulaire circulant de la protéine IRAP («insulin-responsive aminopeptidase»), pour la mise en oeuvre d'une méthode de dosage in *vitro* de la concentration en domaine extracellulaire sécrété de la protéine IRAP dans du sérum ou le plasma d'un mammifère, notamment de l'Homme, ledit dosage étant effectué à l'aide d'un anticorps monoclonal dirigé contre la partie extracellulaire de la protéine IRAP représenté par les SEQ ID NO : 7 à 11.

2. Utilisation du domaine extracellulaire circulant de la protéine IRAP selon la revendication 1, pour le diagnostic et/ou le pronostic in vitro, ou le suivi in vitro de pathologies liées à des défauts de translocation du transporteur du glucose GLUT4 ou de protéines associées.

3. Utilisation du domaine extracellulaire circulant de la protéine IRAP selon la revendication 2, pour le diagnostic et/ou te pronostic in vitro de pathologies associées à la surexpression et/ou à l'augmentation de la translocation vers la membrane de IRAP et/ou de ses isoformes et/ou variants par rapport à un individu sain, ou le suivi in vitro de pathologies associées à la surexpression et/ou à l'augmentation de la translocation vers la membrane de IRAP et/ou de ses isoformes et/ou variants chez un patient, par rapport à un individu sain.

4. Utilisation du domaine extracellulaire circulant de la protéine IRAP selon la revendication 2, pour le diagnostic et/ou le pronostic *in vitro* de pathologies associées à la sous-expression et/ou à la diminution de la translocation vers la membrane de IRAP et/ou de ses isoformes et/ou variants par rapport à un individu sain, ou le suivi *in vitro* de pathologies associées à la sous expression et/ou à la diminution de la translocation vers la membrane de IRAP ou de ses isoformes ou variants chez un patient.

5. Utilisation du domaine extracellulaire circulant de la protéine IRAP selon la revendication 2, pour le diagnostic et/ou le pronostic *in vitro* de la chimiorésistance aux médicaments anticancéreux.

6. Utilisation du domaine extracellulaire circulant de la protéine IRAP l'une quelconque des revendications 1 à 2 ou 4, dans laquelle les pathologies sont parmi celles associées à l'insulino-résistance, le diabète de type 2, le diabète gestationnel, l'hypertension gravidique (prééclampsie) et la menace d'accouchement prématuré.

**7.** Utilisation du domaine extracellulaire circulant de la protéine IRAP et/ou de ses isoformes et/ou variants selon la revendication 1 ou 2, dans laquelle les pathologies sont des maladies prolifératives et notamment les cancers, en particulier ceux dans lesquels IRAP et/ou ses isoformes et/ou variants sont surexprimés et/ou dont translocation vers la membrane est augmentée tels que l'adénocarcinome ovarien, le cancer de l'endomètre, le choriocarcinome, le cancer du pancréas, le cancer du sein, le cancer de la prostate, le cancer de l'estomac, le cancer du rectum ou les cancers de la sphère ORL, les maladies auto-immunes ou inflammatoires.

**8.** Anticorps monoclonal reconnaissant spécifiquement le domaine extracellulaire circulant, ou l'un des épitopes du domaine extracellulaire, de la protéine IRAP (insulin responsive amino peptidase) ledit domaine extracellulaire étant défini par les séquences SEQ ID NO : 7 à 11.

**9.** Anticorps monoclonal selon la revendication 8, le dit anticorps monoclonal étant choisi parmi :

- l'anticorps monoclonal sécrété par l'hybridome déposé à la CNCM (Collection Nationale de Culture de Microorganismes, Institut Pasteur, Paris, France) le 2 juillet 2009, sous le numéro d'accession CNCM I-4181,
- l'anticorps monoclonal sécrété par l'hybridome déposé à la CNCM le 2 juillet 2009, sous le numéro d'accession CNCM I-4182,
- l'anticorps monoclonal sécrété par l'hybridome déposé à la CNCM le 2 juillet 2009, sous le numéro d'accession CNCM I-4183,
- l'anticorps monoclonal sécrété par l'hybridome déposé à la CNCM le 2 juillet 2009, sous le numéro d'accession CNCM I-4184, et
- l'anticorps monoclonal sécrété par l'hybridome déposé à la CNCM le 2 juillet 2009, sous le numéro d'accession CNCM I-4185.

**10.** Anticorps selon la revendication 8 ou 9, ledit anticorps étant marqué avec un composé choisi parmi un radionucléide, un fluorophore, un quantum dot, un marqueur d'enzyme, un substrat d'enzyme, un co-facteur d'enzyme, un inhibiteur d'enzyme ou un haptène.

**11.** Anticorps selon la revendication 8 ou 9, ledit anticorps étant un anticorps humanisé.

**12.** Anticorps monoclonal selon l'une quelconque des revendications 8 à 11, pour une utilisation en tant que médicament, notamment pour le traitement des cancers, en particulier ceux dans lesquels IRAP et/ou ses isoformes et/ou variants sont surexprimés et/ou dont translocation vers la membrane est augmentée tels que l'adénocarcinome ovarien, le cancer de l'endomètre, le choriocarcinome, le cancer du pancréas, le cancer du sein, le cancer de la prostate, le cancer de l'estomac, le cancer du rectum ou les cancers de la sphère ORL, les maladies auto immunes ou inflammatoires, ou pour le traitement de la résistance à la chimiothérapie.

**13.** Hybridome produisant un anticorps selon la revendication 8 ou 9, notamment un hybridome choisi parmi :

a. l'hybridome déposé à la CNCM le 2 juillet 2009, sous le numéro d'accession CNCM I-4181,
b. l'hybridome déposé à la CNCM le 2 juillet 2009, sous le numéro d'accession CNCM I-4182,
c. l'hybridome déposé à la CNCM le 2 juillet 2009, sous le numéro d'accession CNCM I-4183,
d. l'hybridome déposé à la CNCM le 2 juillet 2009, sous le numéro d'accession CNCM I-4184, et
e. l'hybridome déposé à la CNCM le 2 juillet 2009, sous le numéro d'accession CNCM I-4185.

**14.** Procédé de dosage in vitro de la concentration en protéine IRAP , chez un mammifère comprenant une étape de détermination de la concentration du domaine extracellulaire circulant de la protéine IRAP, dans du sérum ou le plasma d'un mammifère, dans lequel le dosage est effectué soit par une méthode immuno-enzymatique soit par une méthode immuno-histochimique, soit par RIA ou IRMA.

**15.** Méthode de diagnostic *in vitro* de pathologies associées à l'insulino-résistance, le diabète de type 2, le diabète gestationnel, l'hypertension gravidique (prééclampsie) et la menace d'accouchement prématuré et comprenant les étapes suivantes :

a. la détermination in vitro de la concentration de domaine extracellulaire circulant de la protéine IRAP, et/ou de l'une de ses isoformes, chez un mammifère à l'aide d'un anticorps selon l'une quelconque des revendications 8 à 11,
b. la comparaison de ladite concentration obtenue à l'étape a. avec celle obtenue in vitro chez un mammifère sain,

c. la déduction à partir de l'étape b. précédente, du fait que le mammifère présente une insulinorésistance, si la concentration obtenue à l'étape a. est inférieure à celle de l'étape b.

16. Méthode de diagnostic *in vitro* de pathologies associées aux cancers, en particulier ceux dans lesquels IRAP, et/ou l'une de ses isoformes, est surexprimée et/ou dont translocation vers la membrane est augmentée tels que l'adénocarcinome ovarien, ou à des maladies auto immunes ou inflammatoires, ou à la résistance à la chimiothérapie et comprenant les étapes suivantes:

a. la détermination in vitro de la concentration de domaine extracellulaire circulant de la protéine IRAP et/ou de ses isoformes et/ou variants chez un mammifère à l'aide d'un anticorps selon l'une quelconque des revendications 8 à 11,
b. la comparaison de ladite concentration obtenue à l'étape a. avec celle obtenue in vitro chez un mammifère contrôle,
c. la déduction à partir de l'étape b. précédente, du fait que le mammifère présente un cancer, si la concentration obtenue à l'étape a. est supérieure à celle de l'étape b.

17. Kit pour la détermination in vitro de la concentration de domaine extracellulaire circulant de la protéine IRAP et/ou de l'une de ses isoformes chez un mammifère comprenant au moins un tampon, et au moins un anticorps selon l'une quelconque des revendications 8 à 11.

18. Kit selon la revendication 17, comprenant également un substrat de la protéine IRAP ainsi qu'un peptide reconnu par l'anticorps.

**Patentansprüche**

1. Verwendung der zirkulierenden extrazellulären Domäne des Proteins IRAP ("insulin-responsive aminopeptidase") zur Durchführung eines Verfahrens der *in* vitro Dosierung der Konzentration an sekretierter extrazellulärer Domäne des Proteins IRAP im Serum oder im Plasma eines Säugetiers, insbesondere des Menschen, wobei die Dosierung mit Hilfe eines monoklonalen Antikörpers erfolgt, der gegen den extrazellulären Anteil des Proteins IRAP gemäß SEQ ID NO:7 bis 11 gerichtet ist.

2. Verwendung der zirkulierenden extrazellulären Domäne des Proteins IRAP nach Anspruch 1 zur *in vitro* Diagnose und/oder Prognose oder der in *vitro* Nachuntersuchung von Krankheiten, die mit Translokationsfehlern des Glukosetransporters GLUT4 oder damit verbundenen Proteinen einhergehen.

3. Verwendung der zirkulierenden extrazellulären Domäne des Proteins IRAP nach Anspruch 2 zur in vitro Diagnose und/oder Prognose von Krankheiten, die mit der Überexpression und/oder Erhöhung der Translokation in Richtung Membran von IRAP und/oder dessen Isoformen und/oder Varianten einhergehen im Vergleich zu einem gesunden Individuum, oder der *in* vitro Nachuntersuchung von Krankheiten, die mit der Überexpression und/oder Erhöhung der Translokation in Richtung Membran von IRAP und/oder dessen Isoformen und/oder Varianten bei einem Patienten einhergehen im Vergleich zu einem gesunden Individuum.

4. Verwendung der zirkulierenden extrazellulären Domäne des Proteins IRAP nach Anspruch 2 zur in vitro Diagnose und/oder Prognose von Krankheiten, die mit der Unterexpression und/oder Verringerung der Translokation in Richtung Membran von IRAP und/oder dessen Isoformen und/oder Varianten einhergehen im Vergleich zu einem gesunden Individuum, oder der in vitro Nachuntersuchung von Krankheiten, die mit der Unterexpression und/oder Verringerung der Translokation in Richtung Membran von IRAP oder dessen Isoformen oder Varianten bei einem Patienten einhergehen.

5. Verwendung der zirkulierenden extrazellulären Domäne des Proteins IRAP nach Anspruch 2 zur *in vitro* Diagnose und/oder Prognose der Chemoresistenz gegenüber Antikrebsarzneimitteln.

6. Verwendung der zirkulierenden extrazellulären Domäne des Proteins IRAP nach einem der Ansprüche 1 bis 2 oder 4, wobei die Krankheiten unter denen sind, die mit Insulinresistenz, Diabetes Typ 2, Schwangerschaftsdiabetes, schwangerschaftsbedingter Hypotonie (Präeklampsie) und der Gefahr einer Frühgeburt einhergehen.

7. Verwendung der zirkulierenden extrazellulären Domäne des Proteins IRAP und/oder dessen Isoformen und/oder Varianten nach Anspruch 1 oder 2, wobei die Krankheiten proliferative Erkrankungen und besonders Krebserkrankungen sind, insbesondere solche, bei denen IRAP und/oder dessen Isoformen und/oder Varianten überexprimiert sind und/oder deren Translokation in Richtung Membran erhöht ist, wie etwa Adenokarzinom der Eierstöcke, Krebs des Endometriums, Choriokarzinom, Pankreaskrebs, Brustkrebs, Prostatakrebs, Magenkrebs, Mastdarmkrebs oder Krebserkrankungen des HNO-Bereichs, Autoimmun- oder Entzündungserkrankungen.

8. Monoklonaler Antikörper, der in spezifischer Weise die zirkulierende extrazelluläre Domäne oder eines der Epitope der extrazellulären Domäne des Proteins IRAP ("insulin-responsive aminopeptidase") erkennt, wobei die extrazelluläre Domäne durch die Sequenzen SEQ ID NO:7 bis 11 definiert ist.

9. Monoklonaler Antikörper nach Anspruch 8, wobei der monoklonale Antikörper ausgewählt ist aus:

- dem monoklonalen Antikörper, der von der Hybridoma sekretiert wird, die bei der CNCM (Collection Nationale de Culture de Microorganismes, Institut Pasteur, Paris, Frankreich) am 2. Juli 2009 unter der Zugangsnummer CNCM 1-4181 hinterlegt wurde,
- dem monoklonalen Antikörper, der von der Hybridoma sekretiert wird, die bei der CNCM am 2. Juli 2009 unter der Zugangsnummer CNCM 1-4182 hinterlegt wurde,
- dem monoklonalen Antikörper, der von der Hybridoma sekretiert wird, die bei der CNCM am 2. Juli 2009 unter der Zugangsnummer CNCM 1-4183 hinterlegt wurde,
- dem monoklonalen Antikörper, der von der Hybridoma sekretiert wird, die bei der CNCM am 2. Juli 2009 unter der Zugangsnummer CNCM 1-4184 hinterlegt wurde, und
- dem monoklonalen Antikörper, der von der Hybridoma sekretiert wird, die bei der CNCM am 2. Juli 2009 unter der Zugangsnummer CNCM 1-4185 hinterlegt wurde.

10. Antikörper nach Anspruch 8 oder 9, wobei der Antikörper mit einer Verbindung markiert ist, die ausgewählt ist aus einem Radionukleid, einem Fluorophor, einem Quantenpunkt, einem Enzymmarker, einem Enzymsubstrat, einem Enzymcofaktor, einem Enzyminhibitor oder einem Hapten.

11. Antikörper nach Anspruch 8 oder 9, wobei der Antikörper ein humanisierter Antikörper ist.

12. Monoklonaler Antikörper nach einem der Ansprüche 8 bis 11 zur Verwendung als Arzneimittel, besonders zur Behandlung von Krebserkrankungen, insbesondere solche, bei denen IRAP und/oder dessen Isoformen und/oder Varianten überexprimiert sind und/oder deren Translokation in Richtung Membran erhöht ist, wie etwa Adenokarzinom der Eierstöcke, Krebs des Endometriums, Choriokarzinom, Pankreaskrebs, Brustkrebs, Prostatakrebs, Magenkrebs, Mastdarmkrebs oder Krebserkrankungen des HNO-Bereichs, Autoimmun- oder Entzündungserkrankungen, oder zur Behandlung der Widerstandsfähigkeit gegenüber der Chemotherapie.

13. Hybridoma, die einen Antikörper nach Anspruch 8 oder 9 produziert, besonders ein Hybridoma ausgewählt aus:

a. der Hybridoma, die bei der CNCM am 2. Juli 2009 unter der Zugangsnummer CNCM I-4181 hinterlegt wurde,
b. der Hybridoma, die bei der CNCM am 2. Juli 2009 unter der Zugangsnummer CNCM 1-4182 hinterlegt wurde,
c. der Hybridoma, die bei der CNCM am 2. Juli 2009 unter der Zugangsnummer CNCM 1-4183 hinterlegt wurde,
d. der Hybridoma, die bei der CNCM am 2. Juli 2009 unter der Zugangsnummer CNCM I-4184 hinterlegt wurde, und
e. der Hybridoma, die bei der CNCM am 2. Juli 2009 unter der Zugangsnummer CNCM 1-4185 hinterlegt wurde.

14. Verfahren der *in vitro* Dosierung der Konzentration des Proteins IRAP bei einem Säugetier, welche einen Schritt der Bestimmung der Konzentration der zirkulierenden extrazellulären Domäne des Proteins IRAP im Serum oder im Plasma eines Säugetiers umfasst, wobei die Dosierung entweder durch ein immunoenzymatisches Verfahren oder ein immunohistochemisches Verfahren erfolgt, entweder durch RIA oder IRMA.

15. Verfahren zur *in vitro* Diagnose von Krankheiten, die mit Insulinresistenz, Diabetes Typ 2, Schwangerschaftsdiabetes, schwangerschaftsbedingter Hypotonie (Präeklampsie) und der Gefahr einer Frühgeburt einhergehen und die folgenden Schritte umfasst:

a. das *in vitro* Bestimmen der Konzentration der zirkulierenden extrazellulären Domäne des Proteins IRAP und/oder eines seiner Isoformen bei einem Säugetier mit Hilfe eines Antikörpers nach einem der Ansprüche 8 bis 11,
b. das Vergleichen der in Schritt a. erhaltenen Konzentration mit jener, die in vitro bei einem gesunden Säugetier erhalten wird,

c. das Rückschließen vom vorhergehenden Schritt b. auf die Tatsache, dass das Säugetier eine Insulinresistenz aufweist, wenn die in Schritt a. erhaltene Konzentration geringer ist, als jene aus Schritt b.

16. Verfahren zur *in vitro* Diagnose von Krankheiten, die mit Krebs einhergehen, insbesondere solche, bei denen IRAP und/oder eines seiner Isoformen überexprimiert ist und/oder deren Translokation in Richtung Membran erhöht ist, wie etwa Adenokarzinom der Eierstöcke öder Autoimmun- oder Entzündungserkrankungen oder Widerstandsfähigkeit gegenüber der Chemotherapie und die folgenden Schritte umfasst:

   a. das Bestimmen der in vitro Konzentration der zirkulierenden extrazellulären Domäne des Proteins IRAP und/oder seiner Isoformen und/oder Varianten bei einem Säugetier mit Hilfe eines Antikörpers nach einem der Ansprüche 8 bis 11,
   b. das Vergleichen der in Schritt a. erhaltenen Konzentration mit jener, die *in vitro* bei einem Kontrollsäugetier erhalten wird,
   c. das Rückschließen vom vorhergehenden Schritt b. auf die Tatsache, dass das Säugetier eine Krebserkrankung aufweist, wenn die in Schritt a, erhaltene Konzentration höher ist, als jene aus Schritt b.

17. Kit zur *in vitro* Bestimmung der Konzentration der zirkulierenden extrazellulären Domäne des Proteins IRAP und/oder eines seiner Isoformen bei einem Säugetier, der wenigstens einen Puffer umfasst und wenigstens einen Antikörper nach einem der Ansprüche 8 bis 11.

18. Kit nach Anspruch 17, der ferner ein Substrat des Proteins IRAP umfasst sowie ein Peptid, das von dem Antikörper erkannt wird.

## Claims

1. Use of the extracellular circulating domain of the IRAP protein ("insulin-responsive aminopeptidase"), for implementing a method for the *in vitro* assay of the concentration of the secreted extracellular domain of the IRAP protein in serum or plasma of a mammal, in particular of Human, said assay being carried out using a monoclonal antibody recognizing the extracellular part of the IRAP protein represented by SEQ ID NO : 7 to 11.

2. Use of the extracellular circulating domain of the IRAP protein according to claim 1, for the *in vitro* diagnosis and/or prognosis, or the in vitro monitoring of pathologies linked to translocation defects of the GLUT4 glucose transporter or associated proteins.

3. Use of the extracellular circulating domain of the IRAP protein according to claim 2, for the *in vitro* diagnosis and/or prognosis of pathologies associated with the overexpression of IRAP at, and/or increase in the translocation to, the membrane of IRAP and/or of its isoforms and/or variants compared with a healthy individual, or the in vitro monitoring of pathologies associated with the overexpression of IRAP at, and/or increase in the translocation to, the plasma membrane of IRAP and/or of its isoforms and/or variants in a patient, compared with a healthy individual.

4. Use of the extracellular circulating domain of the IRAP protein according to claim 2, for the in vitro diagnosis and/or prognosis of pathologies associated with the underexpression of IRAP at, and/or the decrease in the translocation to, the plasma membrane of IRAP and/or its isoforms and/or variants compared with a healthy individual, or the *in vitro* monitoring of pathologies associated with the underexpression of IRAP at, and/or decrease in the translocation to, the plasma membrane of IRAP or its isoforms or variants in a patient.

5. Use of the extracellular circulating domain of the IRAP protein according to claim 2, for the in vitro diagnosis and/or prognosis of chemoresistance to anticancer drugs.

6. Use of the extracellular circulating domain of the IRAP protein according to any one of claims 1 to 2 or 4, in which the pathologies are among those associated with insulin-resistance, type 2 diabetes, gestational diabetes, pregnancy-induced hypertension (preeclampsia) and the risk of premature labour.

7. Use of the extracellular circulating domain of the IRAP protein and/or its isoforms and/or variants according to claim 1 or 2, in which the pathologies are proliferative diseases and in particular cancers, in particular those in which IRAP and/or its isoforms and/or variants are overexpressed and/or translocation of which towards the membrane is increased such as ovarian adenocarcinoma, endometrial cancer, choriocarcinoma, pancreatic cancer, breast cancer, prostate cancer, stomach cancer, rectal cancer or head and neck cancers, auto-immune or inflammatory diseases.

8. Monoclonal antibody specifically recognizing the extracellular circulating domain, or one of the epitopes of the extracellular domain, of the IRAP (insulin-responsive aminopeptidase) protein, said extracellular domain being defined by the sequences SEQ ID NO : 7 to 11.

9. Monoclonal antibody according to claim 8, said monoclonal antibody being chosen from:

- the monoclonal antibody secreted by the hybridoma deposited at the CNCM (Collection Nationale de Culture de Microorganismes, Institut Pasteur, Paris, France) on 2 July 2009, under accession number CNCM I-4181,
- the monoclonal antibody secreted by the hybridoma deposited at the CNCM on 2 July 2009, under accession number CNCM 1-4182,
- the monoclonal antibody secreted by the hybridoma deposited at the CNCM on 2 July 2009, under accession number CNCM 1-4183,
- the monoclonal antibody secreted by the hybridoma deposited at the CNCM on 2 July 2009, under accession number CNCM 1-4184, and
- the monoclonal antibody secreted by the hybridoma deposited at the CNCM on 2 July 2009, under accession number CNCM 1-4185.

10. Antibody according to claim 8 or 9, said antibody being labelled with a compound chosen from a radionucleide, a fluorophore, a quantum dot, an enzyme label, an enzyme substrate, an enzyme cofactor, an enzyme inhibitor or a hapten.

11. Antibody according to claims 8 or 9, said antibody being a humanized antibody.

12. Monoclonal antibody according to any one of claims 8 to 11, for a use as medicament, in particular for the treatment of cancers, in particular those in which IRAP and/or its isoforms and/or variants are overexpressed and/or the translocation of which towards the membrane is increased, such as ovarian adenocarcinoma, endometrial cancer, choriocarcinoma, pancreatic cancer, breast cancer, prostate cancer, stomach cancer, rectal cancer or head and neck cancers, auto-immune or inflammatory diseases, or for the treatment of resistance to chemotherapy.

13. Hybridoma producing an antibody according to claim 8 or 9, in particular a hybridoma chosen from:

a. the hybridoma deposited at the CNCM on 2 July 2009, under accession number CNCM 1-4181,
b. the hybridoma deposited at the CNCM on 2 July 2009, under accession number CNCM 1-4182,
c. the hybridoma deposited at the CNCM on 2 July 2009, under accession number CNCM 1-4183,
d. the hybridoma deposited at the CNCM on 2 July 2009, under accession number CNCM I-4184, and
e. the hybridoma deposited at the CNCM on 2 July 2009, under accession number CNCM 1-4185.

14. Method for the in vitro assay of the concentration of IRAP protein, in a mammal, comprising a step of determination of the concentration of the extracellular circulating domain of the IRAP protein, in serum or plasma of a mammal, in which the assay is carried out either by an immuno-enzymatic method or by an immuno-histochemical method, or by RIA or IRMA.

15. Method for the in vitro diagnosis of pathologies associated with insulin resistance, type 2 diabetes, gestational diabetes, pregnancy-induced hypertension (preeclampsia) and the risk of premature labour and comprising the following steps:

a. the *in* vitro determination of the concentration of extracellular circulating domain of the IRAP protein, and/or one of its isoforms, in a mammal using an antibody according to any one of claims 8 to 11,
b. comparison of said concentration obtained in step a. with that obtained *in vitro* in a healthy mammal,
c. deduction from the previous step b., of the fact that the mammal has insulin-resistance, if the concentration obtained in stage a. is less than that of step b.

16. Method for the in vitro diagnosis of pathologies associated with cancers, in particular those in which IRAP, and/or one of its isoforms, is overexpressed and/or the translocation of which towards the membrane is increased, such as ovarian adenocarcinoma, or autoimmune or inflammatory diseases, or resistance to chemotherapy and comprising the following steps:

a. the *in vitro* determination of the concentration of circulating extracellular domain of the IRAP protein and/or of its isoforms and/or variants in a mammal using an antibody according to any one of claims 8 to 11,
b. comparison of said concentration obtained in stage a. with that obtained *in vitro* in a control mammal,
c. deduction from the previous step b., of the fact that the mammal has a cancer, if the concentration obtained in stage a. is greater than that of step b.

17. Kit for the *in vitro* determination of the concentration of the circulating extracellular domain of the IRAP protein and/or one of its isoforms in a mammal, comprising at least one buffer, and at least one antibody according to any one of claims 8 to 11.

18. Kit according to claim 17, also comprising a substrate of the IRAP protein as well as a peptide recognized by the antibody.

FIGURE 1A

FIGURE 1B

FIGURE 1C

FIGURE 1D

FIGURE 1E

**FIGURE 2**

**FIGURE 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005038462 A

- FR 0803802

**Littérature non-brevet citée dans la description**

- **MIZUTANI, S. ; YOSHINO, M. ; OYA, M.** *Clin Biochem,* 1976, vol. 9 (1), 16-18
- **YAMAHARA, N. ; NOMURA, S. ; SUZUKI, T. ; ITAKURA, A. ; ITO, M. ; OKAMOTO, T. ; TSUJIMOTO, M. ; NAKAZATO, H. ; MIZUTANI, S.** *Life Sci,* 2000, vol. 66 (15), 1401-1410
- **TSUJIMOTO, M. ; MIZUTANI, S. ; ADACHI, H. ; KIMURA, M. ; NAKAZATO, H. ; TOMODA, Y.** *Arch Biochem Biophys,* 1992, vol. 292 (2), 388-392
- **NARUKI, M. ; MIZUTANI, S. ; GOTO, K. ; TSUJIMOTO, M. ; NAKAZATO, H. ; ITAKURA, A. ; MIZUNO, K. ; KURAUCHI, O.; ; KIKKAWA, F. ; TOMODA, Y.** *Peptides,* 1996, vol. 17 (2), 257-261
- **WALLIS, M. G. ; LANKFORD, M. F. ; KELLER, S. R.** *Am J Physiol Endocrinol Metab,* 2007, vol. 293 (4), 1092-1102
- **MATSUMOTO, H. ; ROGI, T. ; YAMASHIRO, K. ; KODAMA, S. ; TSURUOKA, N. ; HATTORI, A. ; TAKIO, K. ; MIZUTANI, S. ; TSUJIMOTO, M.** *Eur J Biochem,* 2000, vol. 267 (1), 46-52
- **ALBISTON, A. L. ; PECK, G. R. ; YEATMAN, H. R. ; FERNANDO, R. ; YE, S. ; CHAI, S. Y.** *Pharmacol Ther,* 2007, vol. 116 (3), 417-427
- **KELLER, S. R. ; SCOTT, H. M. ; MASTICK, C. C. ; AEBERSOLD, R. ; LIENHARD, G. E.** *J Biol Chem,* 1995, vol. 270 (40), 23612-23618
- **ROGI, T. ; TSUJIMOTO, M. ; NAKAZATO, H. ; MIZUTANI, S. ; TOMODA, Y.** *J Biol Chem,* 1996, vol. 271 (1), 56-61
- **ALBISTON, A. L. ; MCDOWALL, S. G. ; MATSACOS, D. ; SIM, P. ; CLUNE, E. ; MUSTAFA, T. ; LEE, J. ; MENDELSOHN, F. A. ; SIMPSON, R. J. ; CONNOLLY, L. M.** *J Biol Chem,* 2001, vol. 276 (52), 48623-48626

- **KELLER, S. R.** *Biol Pharm Bull,* 2004, vol. 27 (6), 761-764
- **KARYLOWSKI, O. ; ZEIGERER, A. ; COHEN, A. ; MCGRAW, T. E.** *Mol Biol Cell,* 2004, vol. 15 (2), 870-882
- **SUBTIL, A. ; LAMPSON, M. A. ; KELLER, S. R. ; MCGRAW, T. E.** *J Biol Chem,* 2000, vol. 275 (7), 4787-4795
- **KELLER, S. R. ; DAVIS, A. C. ; CLAIRMONT, K. B.** *J Biol Chem,* 2002, vol. 277 (20), 17677-17686
- **KAHN, B. B.** *J Clin Invest,* 1992, vol. 89 (5), 1367-1374
- **GARVEY, W. T. ; MAIANU, L. ; ZHU, J. H. ; BRECHTEL-HOOK, G. ; WALLACE, P. ; BARON, A. D.** *J Clin Invest,* 1998, vol. 101 (11), 2377-2386
- **MAIANU, L. ; KELLER, S. R. ; GARVEY, W. T.** *J Clin Endocrinol Metab,* 2001, vol. 86 (11), 5450-5456
- **KONDO C. et al.** *Int. J. Cancer,* 2006, vol. 118, 1390-1394
- **ITO, N. ; NOMURA, S. ; IWASE, A. ; ITO, T. ; KIKKAWA, F. ; TSUJIMOTO, M. ; ISHIURA, S. ; MIZUTANI, S.** *Biochem Biophys Res Commun,* 2004, vol. 314 (4), 1008-1013
- **HOTODA, N. ; KOIKE, H. ; SASAGAWA, N. ; ISHIURA, S.** *Biochem Biophys Res Commun,* 2002, vol. 293 (2), 800-805
- Mammal Species of the World. Johns Hopkins University Press, 16 Novembre 2005
- **KONDO et al.** *Int. J. Cancer,* 2006, vol. 118, 1390-1394
- **YAMAHARA, N. ; NOMURA, S. ; SUZUKI, T. ; ITAKURA, A. ; ITO, M. ; OKAMOTO, T. ; TSUJIMÔTO, M. ; NAKAZATO, H. ; MIZUTANI, S.** *Life Sci,* 2000, vol. 66 (15), 1401-1410

SEQUENCE LISTING

<110> Université Joseph Fourier BOTTARI, Serge

<120> UTILISATION DE LA PROTEINE IRAP POUR LA MISE EN OEUVRE DE METHODES DE DIAGNOSTIC ET DE PRONOSTIQUE

<130> WOB 08 AW UJF IRAP

<150> FR 08/03802
<151> 2004-07-04

<150> FR 08/03802
<151> 2008-07-04

<160> 11

<170> PatentIn version 3.5

<210> 1
<211> 1025
<212> PRT
<213> Homo sapiens

<220>
<221> PEPTIDE
<222> (1)..(1025)

<400> 1

```
Met Glu Pro Phe Thr Asn Asp Arg Leu Gln Leu Pro Arg Asn Met Ile
1               5                   10              15

Glu Asn Ser Met Phe Glu Glu Glu Pro Asp Val Val Asp Leu Ala Lys
            20              25              30

Glu Pro Cys Leu His Pro Leu Glu Pro Asp Glu Val Glu Tyr Glu Pro
            35              40              45

Arg Gly Ser Arg Leu Leu Val Arg Gly Leu Gly Glu His Glu Met Glu
    50              55              60

Glu Asp Glu Glu Asp Tyr Glu Ser Ser Ala Lys Leu Leu Gly Met Ser
65              70              75              80

Phe Met Asn Arg Ser Ser Gly Leu Arg Asn Ser Ala Thr Gly Tyr Arg
            85              90              95

Gln Ser Pro Asp Gly Ala Cys Ser Val Pro Ser Ala Arg Thr Met Val
            100             105             110

Val Cys Ala Phe Val Ile Val Val Ala Val Ser Val Ile Met Val Ile
```

|       | 115   |       |       |       | 120   |       |       |       | 125   |       |       |       |       |       |
|-------|-------|-------|-------|-------|-------|-------|-------|-------|-------|-------|-------|-------|-------|-------|

Tyr Leu Leu Pro Arg Cys Thr Phe Thr Lys Glu Gly Cys His Lys Lys
    130             135             140

Asn Gln Ser Ile Gly Leu Ile Gln Pro Phe Ala Thr Asn Gly Lys Leu
145             150            155             160

Phe Pro Trp Ala Gln Ile Arg Leu Pro Thr Ala Val Val Pro Leu Arg
            165          170             175

Tyr Glu Leu Ser Leu His Pro Asn Leu Thr Ser Met Thr Phe Arg Gly
            180          185          190

Ser Val Thr Ile Ser Val Gln Ala Leu Gln Val Thr Trp Asn Ile Ile
      195          200          205

Leu His Ser Thr Gly His Asn Ile Ser Arg Val Thr Phe Met Ser Ala
    210          215          220

Val Ser Ser Gln Glu Lys Gln Ala Glu Ile Leu Glu Tyr Ala Tyr His
225            230          235          240

Gly Gln Ile Ala Ile Val Ala Pro Glu Ala Leu Leu Ala Gly His Asn
            245          250          255

Tyr Thr Leu Lys Ile Glu Tyr Ser Ala Asn Ile Ser Ser Ser Tyr Tyr
            260          265          270

Gly Phe Tyr Gly Phe Ser Tyr Thr Asp Glu Ser Asn Glu Lys Lys Tyr
            275          280          285

Phe Ala Ala Thr Gln Phe Glu Pro Leu Ala Ala Arg Ser Ala Phe Pro
    290          295          300

Cys Phe Asp Glu Pro Ala Phe Lys Ala Thr Phe Ile Ile Lys Ile Ile
305            310          315          320

Arg Asp Glu Gln Tyr Thr Ala Leu Ser Asn Met Pro Lys Lys Ser Ser
            325          330          335

Val Val Leu Asp Asp Gly Leu Val Gln Asp Glu Phe Ser Glu Ser Val
            340          345          350

Lys Met Ser Thr Tyr Leu Val Ala Phe Ile Val Gly Glu Met Lys Asn
       355           360          365

```
Leu Ser Gln Asp Val Asn Gly Thr Leu Val Ser Ile Tyr Ala Val Pro
    370             375             380

Glu Lys Ile Gly Gln Val His Tyr Ala Leu Glu Thr Thr Val Lys Leu
385             390             395             400

Leu Glu Phe Phe Gln Asn Tyr Phe Glu Ile Gln Tyr Pro Leu Lys Lys
            405             410             415

Leu Asp Leu Val Ala Ile Pro Asp Phe Glu Ala Gly Ala Met Glu Asn
            420             425             430

Trp Gly Leu Leu Thr Phe Arg Glu Glu Thr Leu Leu Tyr Asp Ser Asn
        435             440             445

Thr Ser Ser Met Ala Asp Arg Lys Leu Val Thr Lys Ile Ile Ala His
    450             455             460

Glu Leu Ala His Gln Trp Phe Gly Asn Leu Val Thr Met Lys Trp Trp
465             470             475             480

Asn Asp Leu Trp Leu Asn Glu Gly Phe Ala Thr Phe Met Glu Tyr Phe
            485             490             495

Ser Leu Glu Lys Ile Phe Lys Glu Leu Ser Ser Tyr Glu Asp Phe Leu
            500             505             510

Asp Ala Arg Phe Lys Thr Met Lys Lys Asp Ser Leu Asn Ser Ser His
        515             520             525

Pro Ile Ser Ser Ser Val Gln Ser Ser Glu Gln Ile Glu Glu Met Phe
    530             535             540

Asp Ser Leu Ser Tyr Phe Lys Gly Ser Ser Leu Leu Leu Met Leu Lys
545             550             555             560

Thr Tyr Leu Ser Glu Asp Val Phe Gln His Ala Val Val Leu Tyr Leu
            565             570             575

His Asn His Ser Tyr Ala Ser Ile Gln Ser Asp Asp Leu Trp Asp Ser
        580             585             590

Phe Asn Glu Val Thr Asn Gln Thr Leu Asp Val Lys Arg Met Met Lys
        595             600             605
```

```
Thr Trp Thr Leu Gln Lys Gly Phe Pro Leu Val Thr Val Gln Lys Lys
    610                 615                 620

Gly Lys Glu Leu Phe Ile Gln Gln Glu Arg Phe Phe Leu Asn Met Lys
625                 630                 635                 640

Pro Glu Ile Gln Pro Ser Asp Thr Ser Tyr Leu Trp His Ile Pro Leu
                645                 650                 655

Ser Tyr Val Thr Glu Gly Arg Asn Tyr Ser Lys Tyr Gln Ser Val Ser
                660                 665                 670

Leu Leu Asp Lys Lys Ser Gly Val Ile Asn Leu Thr Glu Glu Val Leu
            675                 680                 685

Trp Val Lys Val Asn Ile Asn Met Asn Gly Tyr Tyr Ile Val His Tyr
    690                 695                 700

Ala Asp Asp Asp Trp Glu Ala Leu Ile His Gln Leu Lys Ile Asn Pro
705                 710                 715                 720

Tyr Val Leu Ser Asp Lys Asp Arg Ala Asn Leu Ile Asn Asn Ile Phe
                725                 730                 735

Glu Leu Ala Gly Leu Gly Lys Val Pro Leu Lys Arg Ala Phe Asp Leu
            740                 745                 750

Ile Asn Tyr Leu Gly Asn Glu Asn His Thr Ala Pro Ile Thr Glu Ala
            755                 760                 765

Leu Phe Gln Thr Asp Leu Ile Tyr Asn Leu Leu Glu Lys Leu Gly Tyr
    770                 775                 780

Met Asp Leu Ala Ser Arg Leu Val Thr Arg Val Phe Lys Leu Leu Gln
785                 790                 795                 800

Asn Gln Ile Gln Gln Gln Thr Trp Thr Asp Glu Gly Thr Pro Ser Met
                805                 810                 815

Arg Glu Leu Arg Ser Ala Leu Leu Glu Phe Ala Cys Thr His Asn Leu
            820                 825                 830

Gly Asn Cys Ser Thr Thr Ala Met Lys Leu Phe Asp Asp Trp Met Ala
            835                 840                 845
```

21

```
          Ser Asn Gly Thr Gln Ser Leu Pro Thr Asp Val Met Thr Thr Val Phe
              850                 855                 860

          Lys Val Gly Ala Lys Thr Asp Lys Gly Trp Ser Phe Leu Leu Gly Lys
          865                 870                 875                 880

          Tyr Ile Ser Ile Gly Ser Glu Ala Glu Lys Asn Lys Ile Leu Glu Ala
                              885                 890                 895

          Leu Ala Ser Ser Glu Asp Val Arg Lys Leu Tyr Trp Leu Met Lys Ser
                      900                 905                 910

          Ser Leu Asn Gly Asp Asn Phe Arg Thr Gln Lys Leu Ser Phe Ile Ile
                  915                 920                 925

          Arg Thr Val Gly Arg His Phe Pro Gly His Leu Leu Ala Trp Asp Phe
              930                 935                 940

          Val Lys Glu Asn Trp Asn Lys Leu Val Gln Lys Phe Pro Leu Gly Ser
          945                 950                 955                 960

          Tyr Thr Ile Gln Asn Ile Val Ala Gly Ser Thr Tyr Leu Phe Ser Thr
                      965                 970                 975

          Lys Thr His Leu Ser Glu Val Gln Ala Phe Phe Glu Asn Gln Ser Glu
                  980                 985                 990

          Ala Thr Phe Arg Leu Arg Cys Val Gln Glu Ala Leu Glu Val Ile Gln
                  995                 1000                1005

          Leu Asn Ile Gln Trp Met Glu Lys Asn Leu Lys Ser Leu Thr Trp
              1010                1015                1020

          Trp Leu
              1025
```

<210> 2
<211> 1011
<212> PRT
<213> Homo sapiens

<220>
<221> PEPTIDE
<222> (1)..(1011)

<400> 2

```
          Met Ile Glu Asn Ser Met Phe Glu Glu Glu Pro Asp Val Val Asp Leu
```

```
         1                  5                      10                        15

         Ala Lys Glu Pro Cys Leu His Pro Leu Glu Pro Asp Glu Val Glu Tyr
                     20                  25              30

         Glu Pro Arg Gly Ser Arg Leu Leu Val Arg Gly Leu Gly Glu His Glu
                     35                  40              45

         Met Glu Glu Asp Glu Glu Asp Tyr Glu Ser Ser Ala Lys Leu Leu Gly
                 50                  55              60

         Met Ser Phe Met Asn Arg Ser Ser Gly Leu Arg Asn Ser Ala Thr Gly
         65                  70                  75                  80

         Tyr Arg Gln Ser Pro Asp Gly Ala Cys Ser Val Pro Ser Ala Arg Thr
                         85                  90                  95

         Met Val Val Cys Ala Phe Val Ile Val Val Ala Val Ser Val Ile Met
                     100                 105                 110

         Val Ile Tyr Leu Leu Pro Arg Cys Thr Phe Thr Lys Glu Gly Cys His
                 115                 120                 125

         Lys Lys Asn Gln Ser Ile Gly Leu Ile Gln Pro Phe Ala Thr Asn Gly
             130                 135                 140

         Lys Leu Phe Pro Trp Ala Gln Ile Arg Leu Pro Thr Ala Val Val Pro
         145                 150                 155                 160

         Leu Arg Tyr Glu Leu Ser Leu His Pro Asn Leu Thr Ser Met Thr Phe
                         165                 170                 175

         Arg Gly Ser Val Thr Ile Ser Val Gln Ala Leu Gln Val Thr Trp Asn
                     180                 185                 190

         Ile Ile Leu His Ser Thr Gly His Asn Ile Ser Arg Val Thr Phe Met
                     195                 200                 205

         Ser Ala Val Ser Ser Gln Glu Lys Gln Ala Glu Ile Leu Glu Tyr Ala
             210                 215                 220

         Tyr His Gly Gln Ile Ala Ile Val Ala Pro Glu Ala Leu Leu Ala Gly
         225                 230                 235                 240

         His Asn Tyr Thr Leu Lys Ile Glu Tyr Ser Ala Asn Ile Ser Ser Ser
                         245                 250                 255
```

```
Tyr Tyr Gly Phe Tyr Gly Phe Ser Tyr Thr Asp Glu Ser Asn Glu Lys
            260             265             270

Lys Tyr Phe Ala Ala Thr Gln Phe Glu Pro Leu Ala Ala Arg Ser Ala
            275             280             285

Phe Pro Cys Phe Asp Glu Pro Ala Phe Lys Ala Thr Phe Ile Ile Lys
            290             295             300

Ile Ile Arg Asp Glu Gln Tyr Thr Ala Leu Ser Asn Met Pro Lys Lys
305             310             315             320

Ser Ser Val Val Leu Asp Asp Gly Leu Val Gln Asp Glu Phe Ser Glu
            325             330             335

Ser Val Lys Met Ser Thr Tyr Leu Val Ala Phe Ile Val Gly Glu Met
            340             345             350

Lys Asn Leu Ser Gln Asp Val Asn Gly Thr Leu Val Ser Ile Tyr Ala
            355             360             365

Val Pro Glu Lys Ile Gly Gln Val His Tyr Ala Leu Glu Thr Thr Val
            370             375             380

Lys Leu Leu Glu Phe Phe Gln Asn Tyr Phe Glu Ile Gln Tyr Pro Leu
385             390             395             400

Lys Lys Leu Asp Leu Val Ala Ile Pro Asp Phe Glu Ala Gly Ala Met
            405             410             415

Glu Asn Trp Gly Leu Leu Thr Phe Arg Glu Glu Thr Leu Leu Tyr Asp
            420             425             430

Ser Asn Thr Ser Ser Met Ala Asp Arg Lys Leu Val Thr Lys Ile Ile
            435             440             445

Ala His Glu Leu Ala His Gln Trp Phe Gly Asn Leu Val Thr Met Lys
            450             455             460

Trp Trp Asn Asp Leu Trp Leu Asn Glu Gly Phe Ala Thr Phe Met Glu
465             470             475             480

Tyr Phe Ser Leu Glu Lys Ile Phe Lys Glu Leu Ser Ser Tyr Glu Asp
            485             490             495
```

Phe Leu Asp Ala Arg Phe Lys Thr Met Lys Lys Asp Ser Leu Asn Ser
500                     505             510

Ser His Pro Ile Ser Ser Ser Val Gln Ser Ser Glu Gln Ile Glu Glu
515             520             525

Met Phe Asp Ser Leu Ser Tyr Phe Lys Gly Ser Ser Leu Leu Leu Met
530             535             540

Leu Lys Thr Tyr Leu Ser Glu Asp Val Phe Gln His Ala Val Val Leu
545             550             555             560

Tyr Leu His Asn His Ser Tyr Ala Ser Ile Gln Ser Asp Asp Leu Trp
565             570             575

Asp Ser Phe Asn Glu Val Thr Asn Gln Thr Leu Asp Val Lys Arg Met
580             585             590

Met Lys Thr Trp Thr Leu Gln Lys Gly Phe Pro Leu Val Thr Val Gln
595             600             605

Lys Lys Gly Lys Glu Leu Phe Ile Gln Gln Glu Arg Phe Phe Leu Asn
610             615             620

Met Lys Pro Glu Ile Gln Pro Ser Asp Thr Ser Tyr Leu Trp His Ile
625             630             635             640

Pro Leu Ser Tyr Val Thr Glu Gly Arg Asn Tyr Ser Lys Tyr Gln Ser
645             650             655

Val Ser Leu Leu Asp Lys Lys Ser Gly Val Ile Asn Leu Thr Glu Glu
660             665             670

Val Leu Trp Val Lys Val Asn Ile Asn Met Asn Gly Tyr Tyr Ile Val
675             680             685

His Tyr Ala Asp Asp Asp Trp Glu Ala Leu Ile His Gln Leu Lys Ile
690             695             700

Asn Pro Tyr Val Leu Ser Asp Lys Asp Arg Ala Asn Leu Ile Asn Asn
705             710             715             720

Ile Phe Glu Leu Ala Gly Leu Gly Lys Val Pro Leu Lys Arg Ala Phe
725             730             735

```
Asp Leu Ile Asn Tyr Leu Gly Asn Glu Asn His Thr Ala Pro Ile Thr
            740                 745               750

Glu Ala Leu Phe Gln Thr Asp Leu Ile Tyr Asn Leu Leu Glu Lys Leu
            755                 760               765

Gly Tyr Met Asp Leu Ala Ser Arg Leu Val Thr Arg Val Phe Lys Leu
            770                 775               780

Leu Gln Asn Gln Ile Gln Gln Gln Thr Trp Thr Asp Glu Gly Thr Pro
785                 790                 795               800

Ser Met Arg Glu Leu Arg Ser Ala Leu Leu Glu Phe Ala Cys Thr His
                805                 810               815

Asn Leu Gly Asn Cys Ser Thr Thr Ala Met Lys Leu Phe Asp Asp Trp
            820                 825               830

Met Ala Ser Asn Gly Thr Gln Ser Leu Pro Thr Asp Val Met Thr Thr
            835                 840                 845

Val Phe Lys Val Gly Ala Lys Thr Asp Lys Gly Trp Ser Phe Leu Leu
    850                 855                 860

Gly Lys Tyr Ile Ser Ile Gly Ser Glu Ala Glu Lys Asn Lys Ile Leu
865                 870                 875               880

Glu Ala Leu Ala Ser Ser Glu Asp Val Arg Lys Leu Tyr Trp Leu Met
                885                 890               895

Lys Ser Ser Leu Asn Gly Asp Asn Phe Arg Thr Gln Lys Leu Ser Phe
                900                 905               910

Ile Ile Arg Thr Val Gly Arg His Phe Pro Gly His Leu Leu Ala Trp
        915                 920               925

Asp Phe Val Lys Glu Asn Trp Asn Lys Leu Val Gln Lys Phe Pro Leu
    930                 935               940

Gly Ser Tyr Thr Ile Gln Asn Ile Val Ala Gly Ser Thr Tyr Leu Phe
945                 950                 955               960

Ser Thr Lys Thr His Leu Ser Glu Val Gln Ala Phe Phe Glu Asn Gln
                965                 970               975

Ser Glu Ala Thr Phe Arg Leu Arg Cys Val Gln Glu Ala Leu Glu Val
```

26

                980                    985                     990

        Ile Gln Leu Asn Ile Gln Trp Met  Glu Lys Asn Leu Lys  Ser Leu Thr
                 995                    1000                  1005


        Trp Trp  Leu
             1010

<210> 3
<211> 1006
<212> PRT
<213> Homo sapiens

<220>
<221> PEPTIDE
<222> (1)..(1006)

<400> 3


        Met Phe Glu Glu Glu Pro Asp Val Val Asp Leu Ala Lys Glu Pro Cys
        1               5               10              15


        Leu His Pro Leu Glu Pro Asp Glu Val Glu Tyr Glu Pro Arg Gly Ser
                 20              25              30


        Arg Leu Leu Val Arg Gly Leu Gly Glu His Glu Met Glu Glu Asp Glu
                 35              40              45


        Glu Asp Tyr Glu Ser Ser Ala Lys Leu Leu Gly Met Ser Phe Met Asn
             50              55              60


        Arg Ser Ser Gly Leu Arg Asn Ser Ala Thr Gly Tyr Arg Gln Ser Pro
        65              70              75              80


        Asp Gly Ala Cys Ser Val Pro Ser Ala Arg Thr Met Val Val Cys Ala
                        85              90              95


        Phe Val Ile Val Val Ala Val Ser Val Ile Met Val Ile Tyr Leu Leu
                 100             105             110


        Pro Arg Cys Thr Phe Thr Lys Glu Gly Cys His Lys Lys Asn Gln Ser
                 115             120             125


        Ile Gly Leu Ile Gln Pro Phe Ala Thr Asn Gly Lys Leu Phe Pro Trp
             130             135             140


        Ala Gln Ile Arg Leu Pro Thr Ala Val Val Pro Leu Arg Tyr Glu Leu
        145             150             155             160

```
Ser Leu His Pro Asn Leu Thr Ser Met Thr Phe Arg Gly Ser Val Thr
            165             170             175

Ile Ser Val Gln Ala Leu Gln Val Thr Trp Asn Ile Ile Leu His Ser
            180             185             190

Thr Gly His Asn Ile Ser Arg Val Thr Phe Met Ser Ala Val Ser Ser
        195             200             205

Gln Glu Lys Gln Ala Glu Ile Leu Glu Tyr Ala Tyr His Gly Gln Ile
    210             215             220

Ala Ile Val Ala Pro Glu Ala Leu Leu Ala Gly His Asn Tyr Thr Leu
225             230             235             240

Lys Ile Glu Tyr Ser Ala Asn Ile Ser Ser Ser Tyr Tyr Gly Phe Tyr
            245             250             255

Gly Phe Ser Tyr Thr Asp Glu Ser Asn Glu Lys Lys Tyr Phe Ala Ala
            260             265             270

Thr Gln Phe Glu Pro Leu Ala Ala Arg Ser Ala Phe Pro Cys Phe Asp
        275             280             285

Glu Pro Ala Phe Lys Ala Thr Phe Ile Ile Lys Ile Ile Arg Asp Glu
    290             295             300

Gln Tyr Thr Ala Leu Ser Asn Met Pro Lys Lys Ser Ser Val Val Leu
305             310             315             320

Asp Asp Gly Leu Val Gln Asp Glu Phe Ser Glu Ser Val Lys Met Ser
            325             330             335

Thr Tyr Leu Val Ala Phe Ile Val Gly Glu Met Lys Asn Leu Ser Gln
            340             345             350

Asp Val Asn Gly Thr Leu Val Ser Ile Tyr Ala Val Pro Glu Lys Ile
        355             360             365

Gly Gln Val His Tyr Ala Leu Glu Thr Thr Val Lys Leu Leu Glu Phe
    370             375             380

Phe Gln Asn Tyr Phe Glu Ile Gln Tyr Pro Leu Lys Lys Leu Asp Leu
385             390             395             400
```

28

```
Val Ala Ile Pro Asp Phe Glu Ala Gly Ala Met Glu Asn Trp Gly Leu
            405                 410                 415

Leu Thr Phe Arg Glu Glu Thr Leu Leu Tyr Asp Ser Asn Thr Ser Ser
            420                 425                 430

Met Ala Asp Arg Lys Leu Val Thr Lys Ile Ile Ala His Glu Leu Ala
            435                 440                 445

His Gln Trp Phe Gly Asn Leu Val Thr Met Lys Trp Trp Asn Asp Leu
    450                 455                 460

Trp Leu Asn Glu Gly Phe Ala Thr Phe Met Glu Tyr Phe Ser Leu Glu
465                 470                 475                 480

Lys Ile Phe Lys Glu Leu Ser Ser Tyr Glu Asp Phe Leu Asp Ala Arg
            485                 490                 495

Phe Lys Thr Met Lys Lys Asp Ser Leu Asn Ser Ser His Pro Ile Ser
            500                 505                 510

Ser Ser Val Gln Ser Ser Glu Gln Ile Glu Glu Met Phe Asp Ser Leu
            515                 520                 525

Ser Tyr Phe Lys Gly Ser Ser Leu Leu Leu Met Leu Lys Thr Tyr Leu
    530                 535                 540

Ser Glu Asp Val Phe Gln His Ala Val Val Leu Tyr Leu His Asn His
545                 550                 555                 560

Ser Tyr Ala Ser Ile Gln Ser Asp Asp Leu Trp Asp Ser Phe Asn Glu
            565                 570                 575

Val Thr Asn Gln Thr Leu Asp Val Lys Arg Met Met Lys Thr Trp Thr
            580                 585                 590

Leu Gln Lys Gly Phe Pro Leu Val Thr Val Gln Lys Lys Gly Lys Glu
            595                 600                 605

Leu Phe Ile Gln Gln Glu Arg Phe Phe Leu Asn Met Lys Pro Glu Ile
    610                 615                 620

Gln Pro Ser Asp Thr Ser Tyr Leu Trp His Ile Pro Leu Ser Tyr Val
625                 630                 635                 640
```

```
Thr Glu Gly Arg Asn Tyr Ser Lys Tyr Gln Ser Val Ser Leu Leu Asp
                645                 650             655

Lys Lys Ser Gly Val Ile Asn Leu Thr Glu Glu Val Leu Trp Val Lys
                660                 665             670

Val Asn Ile Asn Met Asn Gly Tyr Tyr Ile Val His Tyr Ala Asp Asp
            675                 680             685

Asp Trp Glu Ala Leu Ile His Gln Leu Lys Ile Asn Pro Tyr Val Leu
        690                 695             700

Ser Asp Lys Asp Arg Ala Asn Leu Ile Asn Asn Ile Phe Glu Leu Ala
705                 710             715                 720

Gly Leu Gly Lys Val Pro Leu Lys Arg Ala Phe Asp Leu Ile Asn Tyr
                725                 730                 735

Leu Gly Asn Glu Asn His Thr Ala Pro Ile Thr Glu Ala Leu Phe Gln
            740                 745                 750

Thr Asp Leu Ile Tyr Asn Leu Leu Glu Lys Leu Gly Tyr Met Asp Leu
            755                 760                 765

Ala Ser Arg Leu Val Thr Arg Val Phe Lys Leu Leu Gln Asn Gln Ile
    770                 775                 780

Gln Gln Gln Thr Trp Thr Asp Glu Gly Thr Pro Ser Met Arg Glu Leu
785                 790                 795                 800

Arg Ser Ala Leu Leu Glu Phe Ala Cys Thr His Asn Leu Gly Asn Cys
                805                 810                 815

Ser Thr Thr Ala Met Lys Leu Phe Asp Asp Trp Met Ala Ser Asn Gly
            820                 825                 830

Thr Gln Ser Leu Pro Thr Asp Val Met Thr Thr Val Phe Lys Val Gly
            835                 840                 845

Ala Lys Thr Asp Lys Gly Trp Ser Phe Leu Leu Gly Lys Tyr Ile Ser
    850                 855                 860

Ile Gly Ser Glu Ala Glu Lys Asn Lys Ile Leu Glu Ala Leu Ala Ser
865                 870                 875                 880

Ser Glu Asp Val Arg Lys Leu Tyr Trp Leu Met Lys Ser Ser Leu Asn
```

885     890     895

Gly Asp Asn Phe Arg Thr Gln Lys Leu Ser Phe Ile Ile Arg Thr Val
   900     905     910

Gly Arg His Phe Pro Gly His Leu Leu Ala Trp Asp Phe Val Lys Glu
   915     920     925

Asn Trp Asn Lys Leu Val Gln Lys Phe Pro Leu Gly Ser Tyr Thr Ile
   930     935     940

Gln Asn Ile Val Ala Gly Ser Thr Tyr Leu Phe Ser Thr Lys Thr His
945     950     955     960

Leu Ser Glu Val Gln Ala Phe Phe Glu Asn Gln Ser Glu Ala Thr Phe
     965     970     975

Arg Leu Arg Cys Val Gln Glu Ala Leu Glu Val Ile Gln Leu Asn Ile
   980     985     990

Gln Trp Met Glu Lys Asn Leu Lys Ser Leu Thr Trp Trp Leu
   995     1000     1005

<210> 4
<211> 1025
<212> PRT
<213> Homo sapiens

<220>
<221> PEPTIDE
<222> (1)..(1025)

<400> 4

Met Glu Pro Phe Thr Asn Asp Arg Leu Gln Leu Pro Arg Asn Met Ile
1     5     10     15

Glu Asn Ser Met Phe Glu Glu Glu Pro Asp Val Val Asp Leu Ala Lys
   20     25     30

Glu Pro Cys Leu His Pro Leu Glu Pro Asp Glu Val Glu Tyr Glu Pro
   35     40     45

Arg Gly Ser Arg Leu Leu Val Arg Gly Leu Gly Glu His Glu Met Glu
   50     55     60

Glu Asp Glu Glu Asp Tyr Glu Ser Ser Ala Lys Leu Leu Gly Met Ser
65     70     75     80

31

Phe Met Asn Arg Ser Pro Gly Leu Arg Asn Ser Ala Thr Gly Tyr Arg
                    85                    90                    95

Gln Ser Pro Asp Gly Ala Cys Ser Val Pro Ser Ala Arg Thr Met Val
               100                 105                 110

Val Cys Ala Phe Val Ile Val Val Ala Val Ser Val Ile Met Val Ile
          115                 120                 125

Tyr Leu Leu Pro Arg Cys Thr Phe Thr Lys Glu Gly Cys His Lys Lys
    130                 135                 140

Asn Gln Ser Ile Gly Leu Ile Gln Pro Phe Ala Thr Asn Gly Lys Leu
145                 150                 155                 160

Phe Pro Trp Ala Gln Ile Arg Leu Pro Thr Ala Val Val Pro Leu Arg
               165                 170                 175

Tyr Glu Leu Ser Leu His Pro Asn Leu Thr Ser Met Thr Phe Arg Gly
          180                 185                 190

Ser Val Thr Ile Ser Val Gln Ala Leu Gln Val Thr Trp Asn Ile Ile
          195                 200                 205

Leu His Ser Thr Gly His Asn Ile Ser Arg Val Thr Phe Met Ser Ala
    210                 215                 220

Val Ser Ser Gln Glu Lys Gln Ala Glu Ile Leu Glu Tyr Ala Tyr His
225                 230                 235                 240

Gly Gln Ile Ala Ile Val Ala Pro Glu Ala Leu Leu Ala Gly His Asn
               245                 250                 255

Tyr Thr Leu Lys Ile Glu Tyr Ser Ala Asn Ile Ser Ser Ser Tyr Tyr
          260                 265                 270

Gly Phe Tyr Gly Phe Ser Tyr Thr Asp Glu Ser Asn Glu Lys Lys Tyr
          275                 280                 285

Phe Ala Ala Thr Gln Phe Glu Pro Leu Ala Ala Arg Ser Ala Phe Pro
    290                 295                 300

Cys Phe Asp Glu Pro Ala Phe Lys Ala Thr Phe Ile Ile Lys Ile Ile
305                 310                 315                 320

```
Arg Asp Glu Gln Tyr Thr Ala Leu Ser Asn Met Pro Lys Lys Ser Ser
                325                 330             335

Val Val Leu Asp Asp Gly Leu Val Gln Asp Glu Phe Ser Glu Ser Val
            340                 345             350

Lys Met Ser Thr Tyr Leu Val Ala Phe Ile Val Gly Glu Met Lys Asn
            355                 360             365

Leu Ser Gln Asp Val Asn Gly Thr Leu Val Ser Ile Tyr Ala Val Pro
        370                 375             380

Glu Lys Ile Gly Gln Val His Tyr Ala Leu Glu Thr Thr Val Lys Leu
385                 390             395                 400

Leu Glu Phe Phe Gln Asn Tyr Phe Glu Ile Gln Tyr Pro Leu Lys Lys
                405                 410             415

Leu Asp Leu Val Ala Ile Pro Asp Phe Glu Ala Gly Ala Met Glu Asn
            420                 425             430

Trp Gly Leu Leu Thr Phe Arg Glu Glu Thr Leu Leu Tyr Asp Ser Asn
            435                 440             445

Thr Ser Ser Met Ala Asp Arg Lys Leu Val Thr Lys Ile Ile Ala His
    450                 455             460

Glu Leu Ala His Gln Trp Phe Gly Asn Leu Val Thr Met Lys Trp Trp
465                 470             475                 480

Asn Asp Leu Trp Leu Asn Glu Gly Phe Ala Thr Phe Met Glu Tyr Phe
            485                 490             495

Ser Leu Glu Lys Ile Phe Lys Glu Leu Ser Ser Tyr Glu Asp Phe Leu
            500                 505             510

Asp Ala Arg Phe Lys Thr Met Lys Lys Asp Ser Leu Asn Ser Ser His
        515                 520             525

Pro Ile Ser Ser Ser Val Gln Ser Ser Glu Gln Ile Glu Glu Met Phe
    530                 535             540

Asp Ser Leu Ser Tyr Phe Lys Gly Ser Ser Leu Leu Leu Met Leu Lys
545                 550             555             560
```

33

```
Thr Tyr Leu Ser Glu Asp Val Phe Gln His Ala Val Val Leu Tyr Leu
            565             570             575

His Asn His Ser Tyr Ala Ser Ile Gln Ser Asp Asp Leu Trp Asp Ser
        580             585             590

Phe Asn Glu Val Thr Asn Gln Thr Leu Asp Val Lys Arg Met Met Lys
        595             600             605

Thr Trp Thr Leu Gln Lys Gly Phe Pro Leu Val Thr Val Gln Lys Lys
    610             615             620

Gly Lys Glu Leu Phe Ile Gln Gln Glu Arg Phe Phe Leu Asn Met Lys
625             630             635             640

Pro Glu Ile Gln Pro Ser Asp Thr Ser Tyr Leu Trp His Ile Pro Leu
            645             650             655

Ser Tyr Val Thr Glu Gly Arg Asn Tyr Ser Lys Tyr Gln Ser Val Ser
            660             665             670

Leu Leu Asp Lys Lys Ser Gly Val Ile Asn Leu Thr Glu Glu Val Leu
        675             680             685

Trp Val Lys Val Asn Ile Asn Met Asn Gly Tyr Tyr Ile Val His Tyr
    690             695             700

Ala Asp Asp Asp Trp Glu Ala Leu Ile His Gln Leu Lys Ile Asn Pro
705             710             715             720

Tyr Val Leu Ser Asp Lys Asp Arg Ala Asn Leu Ile Asn Asn Ile Phe
            725             730             735

Glu Leu Ala Gly Leu Gly Lys Val Pro Leu Lys Arg Ala Phe Asp Leu
            740             745             750

Ile Asn Tyr Leu Gly Asn Glu Asn His Thr Ala Pro Ile Thr Glu Ala
        755             760             765

Leu Phe Gln Thr Asp Leu Ile Tyr Asn Leu Leu Glu Lys Leu Gly Tyr
    770             775             780

Met Asp Leu Ala Ser Arg Leu Val Thr Arg Val Phe Lys Leu Leu Gln
785             790             795             800

Asn Gln Ile Gln Gln Gln Thr Trp Thr Asp Glu Gly Thr Pro Ser Met
```

```
                    805                      810                      815


        Arg Glu Leu Arg Ser Ala Leu Leu Glu Phe Ala Cys Thr His Asn Leu
                    820                  825                  830


        Gly Asn Cys Ser Thr Thr Ala Met Lys Leu Phe Asp Asp Trp Met Ala
                    835                  840                  845


        Ser Asn Gly Thr Gln Ser Leu Pro Thr Asp Val Met Thr Thr Val Phe
                850                  855                  860


        Lys Val Gly Ala Lys Thr Asp Lys Gly Trp Ser Phe Leu Leu Gly Lys
        865                  870                  875                  880


        Tyr Ile Ser Ile Gly Ser Glu Ala Glu Lys Asn Lys Ile Leu Glu Ala
                        885                  890                  895


        Leu Ala Ser Ser Glu Asp Val Arg Lys Leu Tyr Trp Leu Met Lys Ser
                    900                  905                  910


        Ser Leu Asn Gly Asp Asn Phe Arg Thr Gln Lys Leu Ser Phe Ile Ile
                915                  920                  925


        Arg Thr Val Gly Arg His Phe Pro Gly His Leu Leu Ala Trp Asp Phe
                930                  935                  940


        Val Lys Glu Asn Trp Asn Lys Leu Val Gln Lys Phe Pro Leu Gly Ser
        945                  950                  955                  960


        Tyr Thr Ile Gln Asn Ile Val Ala Gly Ser Thr Tyr Leu Phe Ser Thr
                    965                  970                  975


        Lys Thr His Leu Ser Glu Val Gln Ala Phe Phe Glu Asn Gln Ser Glu
                    980                  985                  990


        Ala Thr Phe Arg Leu Arg Cys Val Gln Glu Ala Leu Glu Val Ile Gln
                    995                  1000                 1005


        Leu Asn Ile Gln Trp Met Glu Lys Asn Leu Lys Ser Leu Thr Trp
                1010                 1015                 1020


        Trp Leu
        1025
```

<210> 5
<211> 1025
<212> PRT

<213> Homo sapiens

<220>
<221> PEPTIDE
<222> (1)..(1025)

<400> 5

```
Met Glu Pro Phe Thr Asn Asp Arg Leu Gln Leu Pro Arg Asn Met Ile
1               5                   10                  15

Glu Asn Ser Met Phe Glu Glu Glu Pro Asp Val Val Asp Leu Ala Lys
            20                  25                  30

Glu Pro Cys Leu His Pro Leu Glu Pro Asp Glu Val Glu Tyr Glu Pro
            35                  40                  45

Arg Gly Ser Arg Leu Leu Val Arg Gly Leu Gly Glu His Glu Met Glu
        50                  55                  60

Glu Asp Glu Glu Asp Tyr Glu Ser Ser Ala Lys Leu Leu Gly Met Ser
65                  70                  75                  80

Phe Met Asn Arg Ser Ser Gly Leu Arg Asn Ser Ala Thr Gly Tyr Arg
                85                  90                  95

Gln Ser Pro Asp Gly Ala Cys Ser Val Pro Ser Ala Arg Thr Met Val
            100                 105                 110

Val Cys Ala Phe Val Ile Val Val Ala Val Ser Val Ile Met Val Ile
        115                 120                 125

Tyr Leu Leu Pro Arg Cys Thr Phe Thr Lys Glu Gly Cys His Lys Lys
    130                 135                 140

Asn Gln Ser Ile Gly Leu Ile Gln Pro Phe Ala Thr Asn Gly Lys Leu
145                 150                 155                 160

Phe Pro Trp Ala Gln Ile Arg Leu Pro Thr Ala Val Val Pro Leu Arg
            165                 170                 175

Tyr Glu Leu Ser Leu His Pro Asn Leu Thr Ser Met Thr Phe Arg Gly
            180                 185                 190

Ser Val Thr Ile Ser Val Gln Ala Leu Gln Val Thr Trp Asn Ile Ile
            195                 200                 205
```

```
Leu His Ser Thr Gly His Asn Ile Ser Arg Val Thr Phe Met Ser Ala
    210             215             220

Val Ser Ser Gln Glu Lys Gln Ala Glu Ile Leu Glu Tyr Ala Tyr His
225             230             235             240

Gly Gln Ile Ala Ile Val Ala Pro Glu Ala Leu Leu Ala Gly His Asn
            245             250             255

Tyr Thr Leu Lys Ile Glu Tyr Ser Ala Asn Ile Ser Ser Ser Tyr Tyr
        260             265             270

Gly Phe Tyr Gly Phe Ser Tyr Thr Asp Glu Ser Asn Glu Lys Lys Tyr
        275             280             285

Phe Ala Ala Thr Gln Phe Glu Pro Leu Ala Ala Arg Ser Ala Phe Pro
    290             295             300

Cys Phe Asp Glu Pro Ala Phe Lys Ala Thr Phe Ile Ile Lys Ile Ile
305             310             315             320

Arg Asp Glu Gln Tyr Thr Ala Leu Ser Asn Met Pro Lys Lys Ser Ser
            325             330             335

Val Val Leu Asp Asp Gly Leu Val Gln Asp Glu Phe Ser Glu Ser Val
        340             345             350

Lys Met Ser Thr Tyr Leu Val Ala Phe Ile Val Gly Glu Met Lys Asn
        355             360             365

Leu Ser Gln Asp Val Asn Gly Thr Leu Val Ser Ile Tyr Ala Val Pro
    370             375             380

Glu Lys Ile Gly Gln Val His Tyr Ala Leu Glu Thr Thr Val Lys Leu
385             390             395             400

Leu Glu Phe Phe Gln Asn Tyr Phe Glu Ile Gln Tyr Pro Leu Lys Lys
        405             410             415

Leu Asp Leu Val Ala Ile Pro Asp Phe Glu Ala Gly Ala Met Glu Asn
        420             425             430

Trp Gly Leu Leu Thr Phe Arg Glu Glu Thr Leu Leu Tyr Asp Ser Asn
        435             440             445
```

```
Thr Ser Ser Met Ala Asp Arg Lys Leu Val Thr Lys Ile Ile Ala His
    450             455             460

Glu Leu Ala His Gln Trp Phe Gly Asn Leu Val Thr Met Lys Trp Trp
465             470             475             480

Asn Asp Leu Trp Leu Asn Glu Gly Phe Ala Thr Phe Met Glu Tyr Phe
            485             490             495

Ser Leu Glu Lys Ile Phe Lys Glu Leu Ser Ser Tyr Glu Asp Phe Leu
            500             505             510

Asp Ala Arg Phe Lys Thr Met Lys Lys Asp Ser Leu Asn Ser Ser His
            515             520             525

Pro Ile Ser Ser Ser Val Gln Ser Ser Glu Gln Ile Glu Glu Met Phe
    530             535             540

Asp Ser Leu Ser Tyr Phe Lys Gly Ser Ser Leu Leu Leu Met Leu Lys
545             550             555             560

Thr Tyr Leu Ser Glu Asp Val Phe Gln His Ala Val Val Leu Tyr Leu
            565             570             575

His Asn His Ser Tyr Ala Ser Ile Gln Ser Asp Asp Leu Trp Asp Ser
            580             585             590

Phe Asn Glu Val Thr Asn Gln Thr Leu Asp Val Lys Arg Met Met Lys
            595             600             605

Thr Trp Thr Leu Gln Lys Gly Phe Pro Leu Val Thr Val Gln Lys Lys
    610             615             620

Gly Lys Glu Leu Phe Ile Gln Gln Glu Arg Phe Phe Leu Asn Met Lys
625             630             635             640

Pro Glu Ile Gln Pro Ser Asp Thr Ser Tyr Leu Trp His Ile Pro Leu
            645             650             655

Ser Tyr Val Thr Glu Gly Arg Asn Tyr Ser Lys Tyr Gln Ser Val Ser
            660             665             670

Leu Leu Asp Lys Lys Ser Gly Val Ile Asn Leu Thr Glu Glu Val Leu
            675             680             685

Trp Val Lys Val Asn Ile Asn Met Asn Gly Tyr Tyr Ile Val His Tyr
```

38

```
             690                        695                         700

    Ala Asp Asp Asp Trp Glu Ala Leu Ile His Gln Leu Lys Ile Asn Pro
    705                 710                 715                 720


    Tyr Val Leu Ser Asp Lys Asp Arg Ala Asn Leu Ile Asn Asn Ile Phe
                    725                 730                 735


    Glu Leu Ala Gly Leu Gly Lys Val Pro Leu Lys Arg Ala Phe Asp Leu
                740                 745                 750


    Ile Asn Tyr Leu Gly Asn Glu Asn His Thr Thr Pro Ile Thr Glu Ala
                755                 760                 765


    Leu Phe Gln Thr Asp Leu Ile Tyr Asn Leu Leu Glu Lys Leu Gly Tyr
        770                 775                 780


    Met Asp Leu Ala Ser Arg Leu Val Thr Arg Val Phe Lys Leu Leu Gln
    785                 790                 795                 800


    Asn Gln Ile Gln Gln Gln Thr Trp Thr Asp Glu Gly Thr Pro Ser Met
                    805                 810                 815


    Arg Glu Leu Arg Ser Ala Leu Leu Glu Phe Ala Cys Thr His Asn Leu
                820                 825                 830


    Gly Asn Cys Ser Thr Thr Ala Met Lys Leu Phe Asp Asp Trp Met Ala
            835                 840                 845


    Ser Asn Gly Thr Gln Ser Leu Pro Thr Asp Val Met Thr Thr Val Phe
        850                 855                 860


    Lys Val Gly Ala Lys Thr Asp Lys Gly Trp Ser Phe Leu Leu Gly Lys
    865                 870                 875                 880


    Tyr Ile Ser Ile Gly Ser Glu Ala Glu Lys Asn Lys Ile Leu Glu Ala
                    885                 890                 895


    Leu Ala Ser Ser Glu Asp Val Arg Lys Leu Tyr Trp Leu Met Lys Ser
                900                 905                 910


    Ser Leu Asn Gly Asp Asn Phe Arg Thr Gln Lys Leu Ser Phe Ile Ile
        915                 920                 925


    Arg Thr Val Gly Arg His Phe Pro Gly His Leu Leu Ala Trp Asp Phe
        930                 935                 940
```

```
Val Lys Glu Asn Trp Asn Lys Leu Val Gln Lys Phe Pro Leu Gly Ser
945                 950                 955                 960

Tyr Thr Ile Gln Asn Ile Val Ala Gly Ser Thr Tyr Leu Phe Ser Thr
                965                 970                 975

Lys Thr His Leu Ser Glu Val Gln Ala Phe Phe Glu Asn Gln Ser Glu
                980                 985                 990

Ala Thr Phe Arg Leu Arg Cys Val Gln Glu Ala Leu Glu Val Ile Gln
            995                 1000                1005

Leu Asn Ile Gln Trp Met Glu Lys Asn Leu Lys Ser Leu Thr Trp
    1010                1015                1020

Trp Leu
    1025
```

<210> 6
<211> 1025
<212> PRT
<213> Homo sapiens

<220>
<221> PEPTIDE
<222> (1)..(1025)

<400> 6

```
Met Glu Pro Phe Thr Asn Asp Arg Leu Gln Leu Pro Arg Asn Met Ile
1               5                   10                  15

Glu Asn Ser Met Phe Glu Glu Glu Pro Asp Val Val Asp Leu Ala Lys
            20                  25                  30

Glu Pro Cys Leu His Pro Leu Glu Pro Asp Glu Val Glu Tyr Glu Pro
            35                  40                  45

Arg Gly Ser Arg Leu Leu Val Arg Gly Leu Gly Glu His Glu Met Glu
    50                  55                  60

Glu Asp Glu Glu Asp Tyr Glu Ser Ser Ala Lys Leu Leu Gly Met Ser
65                  70                  75                  80

Phe Met Asn Arg Ser Ser Gly Leu Arg Asn Ser Ala Thr Gly Tyr Arg
                85                  90                  95
```

```
Gln Ser Pro Asp Gly Ala Cys Ser Val Pro Ser Ala Arg Thr Met Val
        100                 105                 110

Val Cys Ala Phe Val Ile Val Val Ala Val Ser Val Ile Met Val Ile
        115                 120                 125

Tyr Leu Leu Pro Arg Cys Thr Phe Thr Lys Glu Gly Cys His Lys Lys
    130                 135                 140

Asn Gln Ser Ile Gly Leu Ile Gln Pro Phe Ala Thr Asn Gly Lys Leu
145                 150                 155                 160

Phe Pro Trp Ala Gln Ile Arg Leu Pro Thr Ala Val Val Pro Leu Arg
            165                 170                 175

Tyr Glu Leu Ser Leu His Pro Asn Leu Thr Ser Met Thr Phe Arg Gly
            180                 185                 190

Ser Val Thr Ile Ser Val Gln Ala Leu Gln Val Thr Trp Asn Ile Ile
        195                 200                 205

Leu His Ser Thr Gly His Asn Ile Ser Arg Val Thr Phe Met Ser Ala
    210                 215                 220

Val Ser Ser Gln Glu Lys Gln Ala Glu Ile Leu Glu Tyr Ala Tyr His
225                 230                 235                 240

Gly Gln Ile Ala Ile Val Ala Pro Glu Ala Leu Leu Ala Gly His Asn
            245                 250                 255

Tyr Thr Leu Lys Ile Glu Tyr Ser Ala Asn Ile Ser Ser Ser Tyr Tyr
            260                 265                 270

Gly Phe Tyr Gly Phe Ser Tyr Thr Asp Glu Ser Asn Glu Lys Lys Tyr
        275                 280                 285

Phe Ala Ala Thr Gln Phe Glu Pro Leu Ala Ala Arg Ser Ala Phe Pro
    290                 295                 300

Cys Phe Asp Glu Pro Ala Phe Lys Ala Thr Phe Ile Ile Lys Ile Ile
305                 310                 315                 320

Arg Asp Glu Gln Tyr Thr Ala Leu Ser Asn Met Pro Lys Lys Ser Ser
            325                 330                 335
```

41

```
Val Val Leu Asp Asp Gly Leu Val Gln Asp Glu Phe Ser Glu Ser Val
        340                 345                 350

Lys Met Ser Thr Tyr Leu Val Ala Phe Ile Val Gly Glu Met Lys Asn
        355                 360                 365

Leu Ser Gln Asp Val Asn Gly Thr Leu Val Ser Ile Tyr Ala Val Pro
    370                 375                 380

Glu Lys Ile Gly Gln Val His Tyr Ala Leu Glu Thr Thr Val Lys Leu
385                 390                 395                 400

Leu Glu Phe Phe Gln Asn Tyr Phe Glu Ile Gln Tyr Pro Leu Lys Lys
                405                 410                 415

Leu Asp Leu Val Ala Ile Pro Asp Phe Glu Ala Gly Ala Met Glu Asn
        420                 425                 430

Trp Gly Leu Leu Thr Phe Arg Glu Glu Thr Leu Leu Tyr Asp Ser Asn
        435                 440                 445

Thr Ser Ser Met Ala Asp Arg Lys Leu Val Thr Lys Ile Ile Ala His
    450                 455                 460

Glu Leu Ala His Gln Trp Phe Gly Asn Leu Val Thr Met Lys Trp Trp
465                 470                 475                 480

Asn Asp Leu Trp Leu Asn Glu Gly Phe Ala Thr Phe Met Glu Tyr Phe
                485                 490                 495

Ser Leu Glu Lys Ile Phe Lys Glu Leu Ser Ser Tyr Glu Asp Phe Leu
        500                 505                 510

Asp Ala Arg Phe Lys Thr Met Lys Lys Asp Ser Leu Asn Ser Ser His
        515                 520                 525

Pro Ile Ser Ser Ser Val Gln Ser Ser Glu Gln Ile Glu Glu Met Phe
    530                 535                 540

Asp Ser Leu Ser Tyr Phe Lys Gly Ser Ser Leu Leu Leu Met Leu Lys
545                 550                 555                 560

Thr Tyr Leu Ser Glu Asp Val Phe Gln His Ala Val Val Leu Tyr Leu
                565                 570                 575

His Asn His Ser Tyr Ala Ser Ile Gln Ser Asp Asp Leu Trp Asp Ser
```

```
                    580                      585                      590


          Phe Asn Glu Val Thr Asn Gln Thr Leu Asp Val Lys Arg Met Met Lys
                  595                  600                  605


          Thr Trp Thr Leu Gln Lys Gly Phe Pro Leu Val Thr Val Gln Lys Lys
              610                  615                  620


          Gly Lys Glu Leu Phe Ile Gln Gln Glu Arg Phe Phe Leu Asn Met Lys
          625                  630                  635                  640


          Pro Glu Ile Gln Pro Ser Asp Thr Ser Tyr Leu Trp His Ile Pro Leu
                          645                  650                  655


          Ser Tyr Val Thr Glu Gly Arg Asn Tyr Ser Lys Tyr Gln Ser Val Ser
                      660                  665                  670


          Leu Leu Asp Lys Lys Ser Gly Val Ile Asn Leu Thr Glu Glu Val Leu
                  675                  680                  685


          Trp Val Lys Val Asn Ile Asn Met Asn Gly Tyr Tyr Ile Val His Tyr
              690                  695                  700


          Ala Asp Asp Asp Trp Glu Ala Leu Ile His Gln Leu Lys Ile Asn Pro
          705                  710                  715                  720


          Tyr Val Leu Ser Asp Lys Asp Arg Ala Asn Leu Ile Asn Asn Ile Phe
                          725                  730                  735


          Glu Leu Ala Gly Leu Gly Lys Val Pro Leu Lys Arg Ala Phe Asp Leu
                      740                  745                  750


          Ile Asn Tyr Leu Gly Asn Glu Asn His Thr Ala Pro Ile Thr Glu Ala
                      755                  760                  765


          Leu Phe Gln Thr Asp Leu Ile Tyr Asn Leu Leu Glu Lys Leu Gly Tyr
                  770                  775                  780


          Met Asp Leu Ala Ser Arg Leu Val Thr Arg Val Phe Lys Leu Leu Gln
          785                  790                  795                  800


          Asn Gln Ile Gln Gln Gln Thr Trp Thr Asp Glu Gly Thr Pro Ser Met
                          805                  810                  815


          Arg Glu Leu Arg Ser Ala Leu Leu Glu Phe Ala Cys Thr His Asn Leu
                      820                  825                  830
```

```
Gly Asn Cys Ser Thr Thr Ala Met Lys Leu Phe Asp Asp Trp Met Ala
        835                 840             845

Ser Asn Gly Thr Gln Ser Leu Pro Thr Asp Val Met Thr Thr Val Phe
        850                 855             860

Lys Val Gly Ala Lys Thr Asp Lys Gly Trp Ser Phe Leu Leu Gly Lys
865             870                 875                 880

Tyr Ile Ser Ile Gly Ser Glu Ala Glu Lys Asn Lys Ile Leu Glu Ala
            885                 890                 895

Leu Ala Ser Ser Glu Asp Val Arg Lys Leu Tyr Trp Leu Met Lys Ser
        900                 905                 910

Ser Leu Asn Gly Asp Asn Phe Arg Thr Gln Lys Leu Ser Phe Ile Ile
        915                 920                 925

Arg Thr Val Gly Arg His Phe Pro Gly His Leu Leu Ala Trp Asp Phe
    930                 935                 940

Val Lys Glu Asn Trp Asn Lys Leu Val Gln Lys Phe Pro Leu Gly Ser
945             950                 955                 960

Tyr Thr Val Gln Asn Ile Val Ala Gly Ser Thr Tyr Leu Phe Ser Thr
            965                 970                 975

Lys Thr His Leu Ser Glu Val Gln Ala Phe Phe Glu Asn Gln Ser Glu
        980                 985                 990

Ala Thr Phe Arg Leu Arg Cys Val  Gln Glu Ala Leu Glu  Val Ile Gln
        995                 1000                1005

Leu Asn  Ile Gln Trp Met Glu  Lys Asn Leu Lys Ser  Leu Thr Trp
    1010                1015                1020

Trp Leu
    1025
```

<210> 7
<211> 894
<212> PRT
<213> Homo sapiens

<220>
<221> PEPTIDE
<222> (1)..(894)

<400> 7

```
Pro Arg Cys Thr Phe Thr Lys Glu Gly Cys His Lys Lys Asn Gln Ser
1               5               10              15

Ile Gly Leu Ile Gln Pro Phe Ala Thr Asn Gly Lys Leu Phe Pro Trp
            20              25              30

Ala Gln Ile Arg Leu Pro Thr Ala Val Val Pro Leu Arg Tyr Glu Leu
            35              40              45

Ser Leu His Pro Asn Leu Thr Ser Met Thr Phe Arg Gly Ser Val Thr
    50              55              60

Ile Ser Val Gln Ala Leu Gln Val Thr Trp Asn Ile Ile Leu His Ser
65              70              75              80

Thr Gly His Asn Ile Ser Arg Val Thr Phe Met Ser Ala Val Ser Ser
            85              90              95

Gln Glu Lys Gln Ala Glu Ile Leu Glu Tyr Ala Tyr His Gly Gln Ile
            100             105             110

Ala Ile Val Ala Pro Glu Ala Leu Leu Ala Gly His Asn Tyr Thr Leu
        115             120             125

Lys Ile Glu Tyr Ser Ala Asn Ile Ser Ser Ser Tyr Tyr Gly Phe Tyr
    130             135             140

Gly Phe Ser Tyr Thr Asp Glu Ser Asn Glu Lys Lys Tyr Phe Ala Ala
145             150             155             160

Thr Gln Phe Glu Pro Leu Ala Ala Arg Ser Ala Phe Pro Cys Phe Asp
            165             170             175

Glu Pro Ala Phe Lys Ala Thr Phe Ile Ile Lys Ile Ile Arg Asp Glu
        180             185             190

Gln Tyr Thr Ala Leu Ser Asn Met Pro Lys Lys Ser Ser Val Val Leu
    195             200             205

Asp Asp Gly Leu Val Gln Asp Glu Phe Ser Glu Ser Val Lys Met Ser
    210             215             220
```

Thr Tyr Leu Val Ala Phe Ile Val Gly Glu Met Lys Asn Leu Ser Gln
225                 230             235                 240

Asp Val Asn Gly Thr Leu Val Ser Ile Tyr Ala Val Pro Glu Lys Ile
            245             250             255

Gly Gln Val His Tyr Ala Leu Glu Thr Thr Val Lys Leu Leu Glu Phe
            260             265             270

Phe Gln Asn Tyr Phe Glu Ile Gln Tyr Pro Leu Lys Lys Leu Asp Leu
        275             280             285

Val Ala Ile Pro Asp Phe Glu Ala Gly Ala Met Glu Asn Trp Gly Leu
    290             295             300

Leu Thr Phe Arg Glu Glu Thr Leu Leu Tyr Asp Ser Asn Thr Ser Ser
305             310             315             320

Met Ala Asp Arg Lys Leu Val Thr Lys Ile Ile Ala His Glu Leu Ala
            325             330             335

His Gln Trp Phe Gly Asn Leu Val Thr Met Lys Trp Trp Asn Asp Leu
            340             345             350

Trp Leu Asn Glu Gly Phe Ala Thr Phe Met Glu Tyr Phe Ser Leu Glu
        355             360             365

Lys Ile Phe Lys Glu Leu Ser Ser Tyr Glu Asp Phe Leu Asp Ala Arg
    370             375             380

Phe Lys Thr Met Lys Lys Asp Ser Leu Asn Ser Ser His Pro Ile Ser
385             390             395             400

Ser Ser Val Gln Ser Ser Glu Gln Ile Glu Glu Met Phe Asp Ser Leu
            405             410             415

Ser Tyr Phe Lys Gly Ser Ser Leu Leu Leu Met Leu Lys Thr Tyr Leu
        420             425             430

Ser Glu Asp Val Phe Gln His Ala Val Val Leu Tyr Leu His Asn His
        435             440             445

Ser Tyr Ala Ser Ile Gln Ser Asp Asp Leu Trp Asp Ser Phe Asn Glu
    450             455             460

Val Thr Asn Gln Thr Leu Asp Val Lys Arg Met Met Lys Thr Trp Thr

465                    470                    475                    480

Leu Gln Lys Gly Phe Pro Leu Val Thr Val Gln Lys Lys Gly Lys Glu
             485                 490                 495

Leu Phe Ile Gln Gln Glu Arg Phe Phe Leu Asn Met Lys Pro Glu Ile
        500             505            510

Gln Pro Ser Asp Thr Ser Tyr Leu Trp His Ile Pro Leu Ser Tyr Val
        515             520            525

Thr Glu Gly Arg Asn Tyr Ser Lys Tyr Gln Ser Val Ser Leu Leu Asp
        530             535            540

Lys Lys Ser Gly Val Ile Asn Leu Thr Glu Glu Val Leu Trp Val Lys
545             550            555           560

Val Asn Ile Asn Met Asn Gly Tyr Tyr Ile Val His Tyr Ala Asp Asp
            565             570           575

Asp Trp Glu Ala Leu Ile His Gln Leu Lys Ile Asn Pro Tyr Val Leu
        580             585            590

Ser Asp Lys Asp Arg Ala Asn Leu Ile Asn Asn Ile Phe Glu Leu Ala
        595             600            605

Gly Leu Gly Lys Val Pro Leu Lys Arg Ala Phe Asp Leu Ile Asn Tyr
        610             615            620

Leu Gly Asn Glu Asn His Thr Ala Pro Ile Thr Glu Ala Leu Phe Gln
625             630            635           640

Thr Asp Leu Ile Tyr Asn Leu Leu Glu Lys Leu Gly Tyr Met Asp Leu
            645             650           655

Ala Ser Arg Leu Val Thr Arg Val Phe Lys Leu Leu Gln Asn Gln Ile
        660             665            670

Gln Gln Gln Thr Trp Thr Asp Glu Gly Thr Pro Ser Met Arg Glu Leu
        675             680           685

Arg Ser Ala Leu Leu Glu Phe Ala Cys Thr His Asn Leu Gly Asn Cys
        690             695           700

Ser Thr Thr Ala Met Lys Leu Phe Asp Asp Trp Met Ala Ser Asn Gly
705             710            715           720

```
Thr Gln Ser Leu Pro Thr Asp Val Met Thr Thr Val Phe Lys Val Gly
                725             730             735

Ala Lys Thr Asp Lys Gly Trp Ser Phe Leu Leu Gly Lys Tyr Ile Ser
            740             745             750

Ile Gly Ser Glu Ala Glu Lys Asn Lys Ile Leu Glu Ala Leu Ala Ser
        755             760             765

Ser Glu Asp Val Arg Lys Leu Tyr Trp Leu Met Lys Ser Ser Leu Asn
    770             775             780

Gly Asp Asn Phe Arg Thr Gln Lys Leu Ser Phe Ile Ile Arg Thr Val
785             790             795             800

Gly Arg His Phe Pro Gly His Leu Leu Ala Trp Asp Phe Val Lys Glu
            805             810             815

Asn Trp Asn Lys Leu Val Gln Lys Phe Pro Leu Gly Ser Tyr Thr Ile
            820             825             830

Gln Asn Ile Val Ala Gly Ser Thr Tyr Leu Phe Ser Thr Lys Thr His
        835             840             845

Leu Ser Glu Val Gln Ala Phe Phe Glu Asn Gln Ser Glu Ala Thr Phe
    850             855             860

Arg Leu Arg Cys Val Gln Glu Ala Leu Glu Val Ile Gln Leu Asn Ile
865             870             875             880

Gln Trp Met Glu Lys Asn Leu Lys Ser Leu Thr Trp Trp Leu
            885             890
```

<210> 8
<211> 951
<212> PRT
<213> Homo sapiens

<220>
<221> PEPTIDE
<222> (1)..(951)

<400> 8

```
Lys Leu Leu Gly Met Ser Phe Met Asn Arg Ser Ser Gly Leu Arg Asn
1               5               10              15
```

```
Ser Ala Thr Gly Tyr Arg Gln Ser Pro Asp Gly Ala Cys Ser Val Pro
        20                25                30

Ser Ala Arg Thr Met Val Val Cys Ala Phe Val Ile Val Val Ala Val
        35                40                45

Ser Val Ile Met Val Ile Tyr Leu Leu Pro Arg Cys Thr Phe Thr Lys
    50                55                60

Glu Gly Cys His Lys Lys Asn Gln Ser Ile Gly Leu Ile Gln Pro Phe
65                70                75                80

Ala Thr Asn Gly Lys Leu Phe Pro Trp Ala Gln Ile Arg Leu Pro Thr
                85                90                95

Ala Val Val Pro Leu Arg Tyr Glu Leu Ser Leu His Pro Asn Leu Thr
            100                105                110

Ser Met Thr Phe Arg Gly Ser Val Thr Ile Ser Val Gln Ala Leu Gln
        115                120                125

Val Thr Trp Asn Ile Ile Leu His Ser Thr Gly His Asn Ile Ser Arg
    130                135                140

Val Thr Phe Met Ser Ala Val Ser Ser Gln Glu Lys Gln Ala Glu Ile
145                150                155                160

Leu Glu Tyr Ala Tyr His Gly Gln Ile Ala Ile Val Ala Pro Glu Ala
            165                170                175

Leu Leu Ala Gly His Asn Tyr Thr Leu Lys Ile Glu Tyr Ser Ala Asn
            180                185                190

Ile Ser Ser Ser Tyr Tyr Gly Phe Tyr Gly Phe Ser Tyr Thr Asp Glu
        195                200                205

Ser Asn Glu Lys Lys Tyr Phe Ala Ala Thr Gln Phe Glu Pro Leu Ala
    210                215                220

Ala Arg Ser Ala Phe Pro Cys Phe Asp Glu Pro Ala Phe Lys Ala Thr
225                230                235                240

Phe Ile Ile Lys Ile Ile Arg Asp Glu Gln Tyr Thr Ala Leu Ser Asn
            245                250                255
```

```
Met Pro Lys Lys Ser Ser Val Val Leu Asp Asp Gly Leu Val Gln Asp
        260             265             270

Glu Phe Ser Glu Ser Val Lys Met Ser Thr Tyr Leu Val Ala Phe Ile
        275             280             285

Val Gly Glu Met Lys Asn Leu Ser Gln Asp Val Asn Gly Thr Leu Val
        290             295             300

Ser Ile Tyr Ala Val Pro Glu Lys Ile Gly Gln Val His Tyr Ala Leu
305             310             315             320

Glu Thr Thr Val Lys Leu Leu Glu Phe Phe Gln Asn Tyr Phe Glu Ile
            325             330             335

Gln Tyr Pro Leu Lys Lys Leu Asp Leu Val Ala Ile Pro Asp Phe Glu
        340             345             350

Ala Gly Ala Met Glu Asn Trp Gly Leu Leu Thr Phe Arg Glu Glu Thr
        355             360             365

Leu Leu Tyr Asp Ser Asn Thr Ser Ser Met Ala Asp Arg Lys Leu Val
    370             375             380

Thr Lys Ile Ile Ala His Glu Leu Ala His Gln Trp Phe Gly Asn Leu
385             390             395             400

Val Thr Met Lys Trp Trp Asn Asp Leu Trp Leu Asn Glu Gly Phe Ala
            405             410             415

Thr Phe Met Glu Tyr Phe Ser Leu Glu Lys Ile Phe Lys Glu Leu Ser
        420             425             430

Ser Tyr Glu Asp Phe Leu Asp Ala Arg Phe Lys Thr Met Lys Lys Asp
    435             440             445

Ser Leu Asn Ser Ser His Pro Ile Ser Ser Ser Val Gln Ser Ser Glu
    450             455             460

Gln Ile Glu Glu Met Phe Asp Ser Leu Ser Tyr Phe Lys Gly Ser Ser
465             470             475             480

Leu Leu Leu Met Leu Lys Thr Tyr Leu Ser Glu Asp Val Phe Gln His
            485             490             495

Ala Val Val Leu Tyr Leu His Asn His Ser Tyr Ala Ser Ile Gln Ser
```

```
                        500                          505                          510

        Asp Asp Leu Trp Asp Ser Phe Asn Glu Val Thr Asn Gln Thr Leu Asp
                    515                      520                      525

        Val Lys Arg Met Met Lys Thr Trp Thr Leu Gln Lys Gly Phe Pro Leu
            530                      535                      540

        Val Thr Val Gln Lys Lys Gly Lys Glu Leu Phe Ile Gln Gln Glu Arg
        545                          550                      555                  560

        Phe Phe Leu Asn Met Lys Pro Glu Ile Gln Pro Ser Asp Thr Ser Tyr
                        565                      570                      575

        Leu Trp His Ile Pro Leu Ser Tyr Val Thr Glu Gly Arg Asn Tyr Ser
                    580                      585                      590

        Lys Tyr Gln Ser Val Ser Leu Leu Asp Lys Lys Ser Gly Val Ile Asn
                    595                      600                      605

        Leu Thr Glu Glu Val Leu Trp Val Lys Val Asn Ile Asn Met Asn Gly
            610                      615                      620

        Tyr Tyr Ile Val His Tyr Ala Asp Asp Asp Trp Glu Ala Leu Ile His
        625                      630                      635                      640

        Gln Leu Lys Ile Asn Pro Tyr Val Leu Ser Asp Lys Asp Arg Ala Asn
                        645                      650                      655

        Leu Ile Asn Asn Ile Phe Glu Leu Ala Gly Leu Gly Lys Val Pro Leu
                    660                      665                      670

        Lys Arg Ala Phe Asp Leu Ile Asn Tyr Leu Gly Asn Glu Asn His Thr
                    675                      680                      685

        Ala Pro Ile Thr Glu Ala Leu Phe Gln Thr Asp Leu Ile Tyr Asn Leu
            690                      695                      700

        Leu Glu Lys Leu Gly Tyr Met Asp Leu Ala Ser Arg Leu Val Thr Arg
        705                      710                      715                      720

        Val Phe Lys Leu Leu Gln Asn Gln Ile Gln Gln Thr Trp Thr Asp
                        725                      730                      735

        Glu Gly Thr Pro Ser Met Arg Glu Leu Arg Ser Ala Leu Leu Glu Phe
                    740                      745                      750
```

```
Ala Cys Thr His Asn Leu Gly Asn Cys Ser Thr Thr Ala Met Lys Leu
        755                 760             765

Phe Asp Asp Trp Met Ala Ser Asn Gly Thr Gln Ser Leu Pro Thr Asp
        770             775             780

Val Met Thr Thr Val Phe Lys Val Gly Ala Lys Thr Asp Lys Gly Trp
785             790             795                 800

Ser Phe Leu Leu Gly Lys Tyr Ile Ser Ile Gly Ser Glu Ala Glu Lys
            805             810             815

Asn Lys Ile Leu Glu Ala Leu Ala Ser Ser Glu Asp Val Arg Lys Leu
        820             825             830

Tyr Trp Leu Met Lys Ser Ser Leu Asn Gly Asp Asn Phe Arg Thr Gln
        835             840             845

Lys Leu Ser Phe Ile Ile Arg Thr Val Gly Arg His Phe Pro Gly His
    850             855             860

Leu Leu Ala Trp Asp Phe Val Lys Glu Asn Trp Asn Lys Leu Val Gln
865             870             875                 880

Lys Phe Pro Leu Gly Ser Tyr Thr Ile Gln Asn Ile Val Ala Gly Ser
            885             890             895

Thr Tyr Leu Phe Ser Thr Lys Thr His Leu Ser Glu Val Gln Ala Phe
        900             905             910

Phe Glu Asn Gln Ser Glu Ala Thr Phe Arg Leu Arg Cys Val Gln Glu
        915             920             925

Ala Leu Glu Val Ile Gln Leu Asn Ile Gln Trp Met Glu Lys Asn Leu
    930             935             940

Lys Ser Leu Thr Trp Trp Leu
945             950
```

<210> 9
<211> 894
<212> PRT
<213> Homo sapiens

<220>
<221> PEPTIDE
<222> (1)..(894)

52

<400> 9

```
Pro Arg Cys Thr Phe Thr Lys Glu Gly Cys His Lys Lys Asn Gln Ser
1               5                   10              15

Ile Gly Leu Ile Gln Pro Phe Ala Thr Asn Gly Lys Leu Phe Pro Trp
            20              25              30

Ala Gln Ile Arg Leu Pro Thr Ala Val Val Pro Leu Arg Tyr Glu Leu
            35              40              45

Ser Leu His Pro Asn Leu Thr Ser Met Thr Phe Arg Gly Ser Val Thr
            50              55              60

Ile Ser Val Gln Ala Leu Gln Val Thr Trp Asn Ile Ile Leu His Ser
65              70              75              80

Thr Gly His Asn Ile Ser Arg Val Thr Phe Met Ser Ala Val Ser Ser
                85              90              95

Gln Glu Lys Gln Ala Glu Ile Leu Glu Tyr Ala Tyr His Gly Gln Ile
            100             105             110

Ala Ile Val Ala Pro Glu Ala Leu Leu Ala Gly His Asn Tyr Thr Leu
            115             120             125

Lys Ile Glu Tyr Ser Ala Asn Ile Ser Ser Ser Tyr Tyr Gly Phe Tyr
    130             135             140

Gly Phe Ser Tyr Thr Asp Glu Ser Asn Glu Lys Lys Tyr Phe Ala Ala
145             150             155             160

Thr Gln Phe Glu Pro Leu Ala Ala Arg Ser Ala Phe Pro Cys Phe Asp
                165             170             175

Glu Pro Ala Phe Lys Ala Thr Phe Ile Ile Lys Ile Ile Arg Asp Glu
                180             185             190

Gln Tyr Thr Ala Leu Ser Asn Met Pro Lys Lys Ser Ser Val Val Leu
            195             200             205

Asp Asp Gly Leu Val Gln Asp Glu Phe Ser Glu Ser Val Lys Met Ser
    210             215             220
```

```
Thr Tyr Leu Val Ala Phe Ile Val Gly Glu Met Lys Asn Leu Ser Gln
225             230             235                 240

Asp Val Asn Gly Thr Leu Val Ser Ile Tyr Ala Val Pro Glu Lys Ile
                245             250                 255

Gly Gln Val His Tyr Ala Leu Glu Thr Thr Val Lys Leu Leu Glu Phe
                260             265                 270

Phe Gln Asn Tyr Phe Glu Ile Gln Tyr Pro Leu Lys Lys Leu Asp Leu
                275             280                 285

Val Ala Ile Pro Asp Phe Glu Ala Gly Ala Met Glu Asn Trp Gly Leu
290                 295             300

Leu Thr Phe Arg Glu Glu Thr Leu Leu Tyr Asp Ser Asn Thr Ser Ser
305             310                 315                 320

Met Ala Asp Arg Lys Leu Val Thr Lys Ile Ile Ala His Glu Leu Ala
                325             330                 335

His Gln Trp Phe Gly Asn Leu Val Thr Met Lys Trp Trp Asn Asp Leu
                340             345                 350

Trp Leu Asn Glu Gly Phe Ala Thr Phe Met Glu Tyr Phe Ser Leu Glu
                355             360                 365

Lys Ile Phe Lys Glu Leu Ser Ser Tyr Glu Asp Phe Leu Asp Ala Arg
    370                 375                 380

Phe Lys Thr Met Lys Lys Asp Ser Leu Asn Ser Ser His Pro Ile Ser
385                 390                 395                 400

Ser Ser Val Gln Ser Ser Glu Gln Ile Glu Glu Met Phe Asp Ser Leu
                405                 410                 415

Ser Tyr Phe Lys Gly Ser Ser Leu Leu Leu Met Leu Lys Thr Tyr Leu
                420                 425                 430

Ser Glu Asp Val Phe Gln His Ala Val Val Leu Tyr Leu His Asn His
            435                 440                 445

Ser Tyr Ala Ser Ile Gln Ser Asp Asp Leu Trp Asp Ser Phe Asn Glu
    450                 455                 460

Val Thr Asn Gln Thr Leu Asp Val Lys Arg Met Met Lys Thr Trp Thr
```

|     | 465 |     |     |     |     | 470 |     |     |     |     | 475 |     |     |     |     | 480 |
| --- | --- | --- | --- | --- | --- | --- | --- | --- | --- | --- | --- | --- | --- | --- | --- | --- |

Leu Gln Lys Gly Phe Pro Leu Val Thr Val Gln Lys Lys Gly Lys Glu
485 490 495

Leu Phe Ile Gln Gln Glu Arg Phe Phe Leu Asn Met Lys Pro Glu Ile
500 505 510

Gln Pro Ser Asp Thr Ser Tyr Leu Trp His Ile Pro Leu Ser Tyr Val
515 520 525

Thr Glu Gly Arg Asn Tyr Ser Lys Tyr Gln Ser Val Ser Leu Leu Asp
530 535 540

Lys Lys Ser Gly Val Ile Asn Leu Thr Glu Glu Val Leu Trp Val Lys
545 550 555 560

Val Asn Ile Asn Met Asn Gly Tyr Tyr Ile Val His Tyr Ala Asp Asp
565 570 575

Asp Trp Glu Ala Leu Ile His Gln Leu Lys Ile Asn Pro Tyr Val Leu
580 585 590

Ser Asp Lys Asp Arg Ala Asn Leu Ile Asn Asn Ile Phe Glu Leu Ala
595 600 605

Gly Leu Gly Lys Val Pro Leu Lys Arg Ala Phe Asp Leu Ile Asn Tyr
610 615 620

Leu Gly Asn Glu Asn His Thr Ala Pro Ile Thr Glu Ala Leu Phe Gln
625 630 635 640

Thr Asp Leu Ile Tyr Asn Leu Leu Glu Lys Leu Gly Tyr Met Asp Leu
645 650 655

Ala Ser Arg Leu Val Thr Arg Val Phe Lys Leu Leu Gln Asn Gln Ile
660 665 670

Gln Gln Gln Thr Trp Thr Asp Glu Gly Thr Pro Ser Met Arg Glu Leu
675 680 685

Arg Ser Ala Leu Leu Glu Phe Ala Cys Thr His Asn Leu Gly Asn Cys
690 695 700

Ser Thr Thr Ala Met Lys Leu Phe Asp Asp Trp Met Ala Ser Asn Gly
705 710 715 720

55

```
Thr Gln Ser Leu Pro Thr Asp Val Met Thr Thr Val Phe Lys Val Gly
                725             730             735

Ala Lys Thr Asp Lys Gly Trp Ser Phe Leu Leu Gly Lys Tyr Ile Ser
            740             745             750

Ile Gly Ser Glu Ala Glu Lys Asn Lys Ile Leu Glu Ala Leu Ala Ser
        755             760             765

Ser Glu Asp Val Arg Lys Leu Tyr Trp Leu Met Lys Ser Ser Leu Asn
    770             775             780

Gly Asp Asn Phe Arg Thr Gln Lys Leu Ser Phe Ile Ile Arg Thr Val
785             790             795             800

Gly Arg His Phe Pro Gly His Leu Leu Ala Trp Asp Phe Val Lys Glu
            805             810             815

Asn Trp Asn Lys Leu Val Gln Lys Phe Pro Leu Gly Ser Tyr Thr Ile
            820             825             830

Gln Asn Ile Val Ala Gly Ser Thr Tyr Leu Phe Ser Thr Lys Thr His
        835             840             845

Leu Ser Glu Val Gln Ala Phe Phe Glu Asn Gln Ser Glu Ala Thr Phe
    850             855             860

Arg Leu Arg Cys Val Gln Glu Ala Leu Glu Val Ile Gln Leu Asn Ile
865             870             875             880

Gln Trp Met Glu Lys Asn Leu Lys Ser Leu Thr Trp Trp Leu
            885             890
```

<210> 10
<211> 894
<212> PRT
<213> Homo sapiens

<220>
<221> PEPTIDE
<222> (1)..(894)

<400> 10

```
Pro Arg Cys Thr Phe Thr Lys Glu Gly Cys His Lys Lys Asn Gln Ser
1               5               10              15
```

```
Ile Gly Leu Ile Gln Pro Phe Ala Thr Asn Gly Lys Leu Phe Pro Trp
            20                  25                  30

Ala Gln Ile Arg Leu Pro Thr Ala Val Val Pro Leu Arg Tyr Glu Leu
            35                  40                  45

Ser Leu His Pro Asn Leu Thr Ser Met Thr Phe Arg Gly Ser Val Thr
            50                  55                  60

Ile Ser Val Gln Ala Leu Gln Val Thr Trp Asn Ile Ile Leu His Ser
65                  70                  75                  80

Thr Gly His Asn Ile Ser Arg Val Thr Phe Met Ser Ala Val Ser Ser
                85                  90                  95

Gln Glu Lys Gln Ala Glu Ile Leu Glu Tyr Ala Tyr His Gly Gln Ile
            100                 105                 110

Ala Ile Val Ala Pro Glu Ala Leu Leu Ala Gly His Asn Tyr Thr Leu
            115                 120                 125

Lys Ile Glu Tyr Ser Ala Asn Ile Ser Ser Ser Tyr Tyr Gly Phe Tyr
    130                 135                 140

Gly Phe Ser Tyr Thr Asp Glu Ser Asn Glu Lys Lys Tyr Phe Ala Ala
145                 150                 155                 160

Thr Gln Phe Glu Pro Leu Ala Ala Arg Ser Ala Phe Pro Cys Phe Asp
            165                 170                 175

Glu Pro Ala Phe Lys Ala Thr Phe Ile Ile Lys Ile Ile Arg Asp Glu
            180                 185                 190

Gln Tyr Thr Ala Leu Ser Asn Met Pro Lys Lys Ser Ser Val Val Leu
    195                 200                 205

Asp Asp Gly Leu Val Gln Asp Glu Phe Ser Glu Ser Val Lys Met Ser
    210                 215                 220

Thr Tyr Leu Val Ala Phe Ile Val Gly Glu Met Lys Asn Leu Ser Gln
225                 230                 235                 240

Asp Val Asn Gly Thr Leu Val Ser Ile Tyr Ala Val Pro Glu Lys Ile
                245                 250                 255
```

57

Gly Gln Val His Tyr Ala Leu Glu Thr Thr Val Lys Leu Leu Glu Phe
        260                 265                 270

Phe Gln Asn Tyr Phe Glu Ile Gln Tyr Pro Leu Lys Lys Leu Asp Leu
        275                 280                 285

Val Ala Ile Pro Asp Phe Glu Ala Gly Ala Met Glu Asn Trp Gly Leu
        290                 295                 300

Leu Thr Phe Arg Glu Glu Thr Leu Leu Tyr Asp Ser Asn Thr Ser Ser
305                 310                 315                 320

Met Ala Asp Arg Lys Leu Val Thr Lys Ile Ile Ala His Glu Leu Ala
            325                 330                 335

His Gln Trp Phe Gly Asn Leu Val Thr Met Lys Trp Trp Asn Asp Leu
        340                 345                 350

Trp Leu Asn Glu Gly Phe Ala Thr Phe Met Glu Tyr Phe Ser Leu Glu
        355                 360                 365

Lys Ile Phe Lys Glu Leu Ser Ser Tyr Glu Asp Phe Leu Asp Ala Arg
    370                 375                 380

Phe Lys Thr Met Lys Lys Asp Ser Leu Asn Ser Ser His Pro Ile Ser
385                 390                 395                 400

Ser Ser Val Gln Ser Ser Glu Gln Ile Glu Glu Met Phe Asp Ser Leu
            405                 410                 415

Ser Tyr Phe Lys Gly Ser Ser Leu Leu Leu Met Leu Lys Thr Tyr Leu
        420                 425                 430

Ser Glu Asp Val Phe Gln His Ala Val Val Leu Tyr Leu His Asn His
        435                 440                 445

Ser Tyr Ala Ser Ile Gln Ser Asp Asp Leu Trp Asp Ser Phe Asn Glu
    450                 455                 460

Val Thr Asn Gln Thr Leu Asp Val Lys Arg Met Met Lys Thr Trp Thr
465                 470                 475                 480

Leu Gln Lys Gly Phe Pro Leu Val Thr Val Gln Lys Lys Gly Lys Glu
            485                 490                 495

Leu Phe Ile Gln Gln Glu Arg Phe Phe Leu Asn Met Lys Pro Glu Ile

                    500                        505                        510

        Gln Pro Ser Asp Thr Ser Tyr Leu Trp His Ile Pro Leu Ser Tyr Val
              515                  520                  525

        Thr Glu Gly Arg Asn Tyr Ser Lys Tyr Gln Ser Val Ser Leu Leu Asp
          530                  535                  540

        Lys Lys Ser Gly Val Ile Asn Leu Thr Glu Glu Val Leu Trp Val Lys
        545                  550                  555                  560

        Val Asn Ile Asn Met Asn Gly Tyr Tyr Ile Val His Tyr Ala Asp Asp
                      565                  570                  575

        Asp Trp Glu Ala Leu Ile His Gln Leu Lys Ile Asn Pro Tyr Val Leu
                  580                  585                  590

        Ser Asp Lys Asp Arg Ala Asn Leu Ile Asn Asn Ile Phe Glu Leu Ala
              595                  600                  605

        Gly Leu Gly Lys Val Pro Leu Lys Arg Ala Phe Asp Leu Ile Asn Tyr
          610                  615                  620

        Leu Gly Asn Glu Asn His Thr Thr Pro Ile Thr Glu Ala Leu Phe Gln
        625                  630                  635                  640

        Thr Asp Leu Ile Tyr Asn Leu Leu Glu Lys Leu Gly Tyr Met Asp Leu
                      645                  650                  655

        Ala Ser Arg Leu Val Thr Arg Val Phe Lys Leu Leu Gln Asn Gln Ile
                  660                  665                  670

        Gln Gln Gln Thr Trp Thr Asp Glu Gly Thr Pro Ser Met Arg Glu Leu
              675                  680                  685

        Arg Ser Ala Leu Leu Glu Phe Ala Cys Thr His Asn Leu Gly Asn Cys
          690                  695                  700

        Ser Thr Thr Ala Met Lys Leu Phe Asp Asp Trp Met Ala Ser Asn Gly
        705                  710                  715                  720

        Thr Gln Ser Leu Pro Thr Asp Val Met Thr Thr Val Phe Lys Val Gly
                      725                  730                  735

        Ala Lys Thr Asp Lys Gly Trp Ser Phe Leu Leu Gly Lys Tyr Ile Ser
                  740                  745                  750

```
Ile Gly Ser Glu Ala Glu Lys Asn Lys Ile Leu Glu Ala Leu Ala Ser
        755             760             765

Ser Glu Asp Val Arg Lys Leu Tyr Trp Leu Met Lys Ser Ser Leu Asn
        770             775             780

Gly Asp Asn Phe Arg Thr Gln Lys Leu Ser Phe Ile Ile Arg Thr Val
785             790             795             800

Gly Arg His Phe Pro Gly His Leu Leu Ala Trp Asp Phe Val Lys Glu
                805             810             815

Asn Trp Asn Lys Leu Val Gln Lys Phe Pro Leu Gly Ser Tyr Thr Ile
                820             825             830

Gln Asn Ile Val Ala Gly Ser Thr Tyr Leu Phe Ser Thr Lys Thr His
        835             840             845

Leu Ser Glu Val Gln Ala Phe Phe Glu Asn Gln Ser Glu Ala Thr Phe
    850             855             860

Arg Leu Arg Cys Val Gln Glu Ala Leu Glu Val Ile Gln Leu Asn Ile
865             870             875             880

Gln Trp Met Glu Lys Asn Leu Lys Ser Leu Thr Trp Trp Leu
                885             890
```

<210> 11
<211> 894
<212> PRT
<213> Homo sapiens

<220>
<221> PEPTIDE
<222> (1)..(894)

<400> 11

```
Pro Arg Cys Thr Phe Thr Lys Glu Gly Cys His Lys Lys Asn Gln Ser
1               5               10              15

Ile Gly Leu Ile Gln Pro Phe Ala Thr Asn Gly Lys Leu Phe Pro Trp
            20              25              30

Ala Gln Ile Arg Leu Pro Thr Ala Val Val Pro Leu Arg Tyr Glu Leu
        35              40              45
```

```
Ser Leu His Pro Asn Leu Thr Ser Met Thr Phe Arg Gly Ser Val Thr
    50              55              60

Ile Ser Val Gln Ala Leu Gln Val Thr Trp Asn Ile Ile Leu His Ser
65              70              75                  80

Thr Gly His Asn Ile Ser Arg Val Thr Phe Met Ser Ala Val Ser Ser
                85              90                  95

Gln Glu Lys Gln Ala Glu Ile Leu Glu Tyr Ala Tyr His Gly Gln Ile
            100             105             110

Ala Ile Val Ala Pro Glu Ala Leu Leu Ala Gly His Asn Tyr Thr Leu
        115             120             125

Lys Ile Glu Tyr Ser Ala Asn Ile Ser Ser Ser Tyr Tyr Gly Phe Tyr
    130             135             140

Gly Phe Ser Tyr Thr Asp Glu Ser Asn Glu Lys Lys Tyr Phe Ala Ala
145             150             155             160

Thr Gln Phe Glu Pro Leu Ala Ala Arg Ser Ala Phe Pro Cys Phe Asp
            165             170             175

Glu Pro Ala Phe Lys Ala Thr Phe Ile Ile Lys Ile Ile Arg Asp Glu
            180             185             190

Gln Tyr Thr Ala Leu Ser Asn Met Pro Lys Lys Ser Ser Val Val Leu
    195             200             205

Asp Asp Gly Leu Val Gln Asp Glu Phe Ser Glu Ser Val Lys Met Ser
    210             215             220

Thr Tyr Leu Val Ala Phe Ile Val Gly Glu Met Lys Asn Leu Ser Gln
225             230             235             240

Asp Val Asn Gly Thr Leu Val Ser Ile Tyr Ala Val Pro Glu Lys Ile
            245             250             255

Gly Gln Val His Tyr Ala Leu Glu Thr Thr Val Lys Leu Leu Glu Phe
            260             265             270

Phe Gln Asn Tyr Phe Glu Ile Gln Tyr Pro Leu Lys Lys Leu Asp Leu
            275             280             285
```

Val Ala Ile Pro Asp Phe Glu Ala Gly Ala Met Glu Asn Trp Gly Leu
290                         295                     300

Leu Thr Phe Arg Glu Glu Thr Leu Leu Tyr Asp Ser Asn Thr Ser Ser
305                         310                     315                     320

Met Ala Asp Arg Lys Leu Val Thr Lys Ile Ile Ala His Glu Leu Ala
325                         330                     335

His Gln Trp Phe Gly Asn Leu Val Thr Met Lys Trp Trp Asn Asp Leu
340                         345                     350

Trp Leu Asn Glu Gly Phe Ala Thr Phe Met Glu Tyr Phe Ser Leu Glu
355                         360                     365

Lys Ile Phe Lys Glu Leu Ser Ser Tyr Glu Asp Phe Leu Asp Ala Arg
370                         375                     380

Phe Lys Thr Met Lys Lys Asp Ser Leu Asn Ser Ser His Pro Ile Ser
385                         390                     395                     400

Ser Ser Val Gln Ser Ser Glu Gln Ile Glu Glu Met Phe Asp Ser Leu
405                         410                     415

Ser Tyr Phe Lys Gly Ser Ser Leu Leu Leu Met Leu Lys Thr Tyr Leu
420                         425                     430

Ser Glu Asp Val Phe Gln His Ala Val Val Leu Tyr Leu His Asn His
435                         440                     445

Ser Tyr Ala Ser Ile Gln Ser Asp Asp Leu Trp Asp Ser Phe Asn Glu
450                         455                     460

Val Thr Asn Gln Thr Leu Asp Val Lys Arg Met Met Lys Thr Trp Thr
465                         470                     475                     480

Leu Gln Lys Gly Phe Pro Leu Val Thr Val Gln Lys Lys Gly Lys Glu
485                         490                     495

Leu Phe Ile Gln Gln Glu Arg Phe Phe Leu Asn Met Lys Pro Glu Ile
500                         505                     510

Gln Pro Ser Asp Thr Ser Tyr Leu Trp His Ile Pro Leu Ser Tyr Val
515                         520                     525

Thr Glu Gly Arg Asn Tyr Ser Lys Tyr Gln Ser Val Ser Leu Leu Asp

530                     535                         540

Lys Lys Ser Gly Val Ile Asn Leu Thr Glu Glu Val Leu Trp Val Lys
545             550             555             560

Val Asn Ile Asn Met Asn Gly Tyr Tyr Ile Val His Tyr Ala Asp Asp
            565             570             575

Asp Trp Glu Ala Leu Ile His Gln Leu Lys Ile Asn Pro Tyr Val Leu
        580             585             590

Ser Asp Lys Asp Arg Ala Asn Leu Ile Asn Asn Ile Phe Glu Leu Ala
        595             600             605

Gly Leu Gly Lys Val Pro Leu Lys Arg Ala Phe Asp Leu Ile Asn Tyr
        610             615             620

Leu Gly Asn Glu Asn His Thr Ala Pro Ile Thr Glu Ala Leu Phe Gln
625             630             635             640

Thr Asp Leu Ile Tyr Asn Leu Leu Glu Lys Leu Gly Tyr Met Asp Leu
            645             650             655

Ala Ser Arg Leu Val Thr Arg Val Phe Lys Leu Leu Gln Asn Gln Ile
        660             665             670

Gln Gln Gln Thr Trp Thr Asp Glu Gly Thr Pro Ser Met Arg Glu Leu
        675             680             685

Arg Ser Ala Leu Leu Glu Phe Ala Cys Thr His Asn Leu Gly Asn Cys
        690             695             700

Ser Thr Thr Ala Met Lys Leu Phe Asp Asp Trp Met Ala Ser Asn Gly
705             710             715             720

Thr Gln Ser Leu Pro Thr Asp Val Met Thr Thr Val Phe Lys Val Gly
            725             730             735

Ala Lys Thr Asp Lys Gly Trp Ser Phe Leu Leu Gly Lys Tyr Ile Ser
        740             745             750

Ile Gly Ser Glu Ala Glu Lys Asn Lys Ile Leu Glu Ala Leu Ala Ser
        755             760             765

Ser Glu Asp Val Arg Lys Leu Tyr Trp Leu Met Lys Ser Ser Leu Asn
        770             775             780

```
Gly Asp Asn Phe Arg Thr Gln Lys Leu Ser Phe Ile Ile Arg Thr Val
785                 790             795                 800

Gly Arg His Phe Pro Gly His Leu Leu Ala Trp Asp Phe Val Lys Glu
            805             810                 815

Asn Trp Asn Lys Leu Val Gln Lys Phe Pro Leu Gly Ser Tyr Thr Val
            820             825             830

Gln Asn Ile Val Ala Gly Ser Thr Tyr Leu Phe Ser Thr Lys Thr His
        835             840                 845

Leu Ser Glu Val Gln Ala Phe Phe Glu Asn Gln Ser Glu Ala Thr Phe
    850                 855             860

Arg Leu Arg Cys Val Gln Glu Ala Leu Glu Val Ile Gln Leu Asn Ile
865                 870                 875                 880

Gln Trp Met Glu Lys Asn Leu Lys Ser Leu Thr Trp Trp Leu
            885             890
```